# EUROPEAN PATENT APPLICATION

(11) **EP 4 183 408 A1**
(43) Date of publication of application: **24.05.2023**
(21) Application number: 22203212.0
(22) Date of filing: 18.09.2015
(51) Int. Cl.: A61K 39/00, A61K 39/02, A61K 39/385

(54) **COMBINED ENTEROTOXIGENIC ESCHERICHIA COLI AND SHIGELLA RECOMBINANT**

(30) Priority: 24.09.2014 US 201462054454 P; 05.11.2014 US 201462075399 P; 04.03.2015 US 201562127927 P; 04.03.2015 US 201562127935 P; 22.05.2015 US 201562165301 P; 08.06.2015 US 201514733114
(62) Divisional of application: 15845465.2
(71) Applicant: The United States of America as Represented by The Secretary of the Navy, Silver Spring, MD 20910-7500 (US)
(72) Inventor: GUERRY, Patricia, Silver Spring, 20906 (US); SAVARINO, Stephen, Kensington, 20895 (US); MONTEIRO, Mario, Arthur, Guelph, N1E7A5 (CA)
(74) Representative: Cabinet Beau de Loménie

(57) **Abstract**

The inventive subject matter relates to a construct comprising antigens derived from multiple enterobacteria including Campylobacter jejuni capsule polysaccharide polymer, enterotoxigenic Escherichia coli recombinant polypeptide construct and lipopolysaccharide from Shigella spp.. The subject invention also relates to a method of inducing an immune response utilizing the inventive composition.

## Description

### CROSS-REFERENCES TO RELATED APPLICATIONS

This application is a Continuation-in-Part to U.S. Nonprovisional application 11/340,003, filed January 10, 2006, which claims priority to U.S. Provisional application 60/642,771 filed January 11, 2005, and a Continuation-in-Part to U.S. Nonprovisional application 11/524,057 filed September 20, 2006, which claims priority to U.S. Provisional application 60/722,086, filed September 21, 2005, and a Continuation-in-Part to U.S. Nonprovisional Application 14/048,264, filed October 8, 2013, which claims priority to U.S. Provisional application 61/727,943, filed November 19, 2012, the contents of which are herein incorporated by reference. This application also claims priority to U.S. Provisional application 62/054,454, filed 24 September 2014, U.S. Provisional application 62/127,927, filed March 4, 2015, U.S. Provisional application 62/165,301, filed May 22, 2015, U.S. Provisional application 62/127,935, filed March 4, 2015, and U.S. Provisional application 62/075,399, filed November 5, 2014, the contents of which are herein incorporated by reference.

### BACKGROUND OF INVENTION

### Field of the Invention

The inventive subject matter relates to a recombinant construct against enterotoxigenic *Escherichia coli* and *Campylobacter jejuni* comprising a combined anti-ETEC recombinant polypeptide construct and *C. jejuni* campsule polysaccharide.

### Description of Related Art

Enterotoxigenic *Escherichia coli* (ETEC), *Shigella,* spp. and *Campylobacter jejuni* (CJ) are major causes of bacterial diarrhea worldwide. Both pathogens are a serious health threat to western travelers and young children in resource-limited countries, making them apt target populations for a single or dual pathogen vaccine against ETEC and CJ. No FDA-licensed vaccines are available for either pathogen.

ETEC causes an estimated 210 million cases of diarrhea and 380,000 deaths annually among infants and young children. Moreover, ETEC is the most common cause of travelers' diarrhea. ETEC causes diarrhea ranging in severity from mild illness to severe cholera-like purging. There are two major virulence factors, adhesive fimbriae, dubbed colonization factors (CFs), and enterotoxins. Surface-expressed CFs, consisting of complex protein heteropolymers, mediate adherence to the small intestinal epithelium to initiate colonization within this privileged host niche. ETEC produce one or both of two different enterotoxins, a heat-labile (LT) and a heat-stable enterotoxin (STI). LT and STI intoxicate epithelial cells, resulting in fluid and electrolyte secretion and clinical diarrhea. LT is highly immunogenic and a potent adjuvant, while STI is a small, poorly immunogenic peptide.

Prevalent CFs and a non-toxic form of the LT (or its congener cholera toxin (CT)) have been the focus for several strategies to develop an ETEC vaccine. Such antigens have been used individually or bundled as components of a whole-cell killed vaccine, live vaccines vectored by attenuated ETEC or other enterobacterial species (e.g., *Shigella* and *Vibrio cholerae* 01), and purified protein vaccines. None has yet been shown to confer sufficiently high and broad levels of protection. The weight of evidence from clinical trials indicates that anti-LT immunity confers short-term protection against LT-producing ETEC. There is also evidence to show that certain CFs function as protective antigens. There are, however, significant challenges for ETEC vaccine development. For one, about half of all ETEC express only STI, for which anti-LT immunity is not thought to be effective, thus necessitating anti-CF or anti-bacterial immunity. Also, the diversity of ETEC CFs poses issues for achievement of sufficiently broad coverage with inclusion of a realistic number of CFs.

### SUMMARY OF THE INVENTION

The invention relates to an immunogenic construct comprising a polypeptide construct expressing enterotoxigenic *Escherichia coli* (ETEC) fimbrial subunits combined with a *Campylobacter jejuni* capsule polysaccharide or *Shigella* spp lipopolysaccharide (LPS).

In a preferred embodiment, one or more *Camplobacter jejuni* capsule polysaccharides are conjugated to one or more Escherichia coli enterotoxigenic recombinant polypeptide constructs. In another embodiment, *Shigella* LPS is conjugated to the ETEC polypeptide construct.

*Campylobacger jejuni* is associated with induction of Guillain-Barre Syndrome (GBS), a post-infectious polyneuropathy that can result in paralysis. The association is due to molecular mimicry between the sialic acid containining-outer core of the lipooligosaccharide (LOS) and human gangliosides (5, 6, 89, 91). Thus, antibodies generated against LOS cores result in an autoimmune response to human neural tissue. Use of capsule polysaccharide from *C. jejuni* can induce an immune response without the possible induction of Guillain-Barre Syndrome.

In a preferred embodiment, the composition comprises an ETEC recombinant polypeptide construct design wherein major or minor subunits, derived from the same ETEC fimbrial type, are connected, via polypeptide linkers, and stabilized by donor strand complementation. The C-terminal most ETEC major subunit is connected, via a linker, to a donor strand region from an ETEC major subunit, which can be either homologous or heterologous to the C-terminal major subunit. The immunogenic composition can comprise a whole or an immunogenic fragment, containing a donor β strand region, of the ETEC fimbrial major or minor subunits. In some construct examples, in order to avoid inadvertent association of subunits, especially in CS6 subunits to each other, major ETEC fimbrial subunits can contain an N-terminal deletion of 14 to 18 amino acids.

In another embodiment one or more of the above constructs are connected, via a polypeptide linker, to form a multipartite fusion construct, wherein the subunits derived from multiple fimbrial types are expressed. In this embodiment, the fimbrial subunits can be derived from any ETEC fimbrial type, including, but not limited to: ETEC class 5 fimbriae type, including class 5a, 5b or 5c; ETEC CS3; and ETEC CS6.

The embodied multipartite construct can contain a deletion of the N-terminal region of one or more fimbrial subunits to avoid undesirable associations with other monomers or multimers and to remove reduce amino acid sequence length between polypeptides to reduce the protease cleavage.

DNA encoding the ETEC recombinant polypeptide construct can be used to express a polypeptide for attachment to *C. jejuni* or *Shigella* LPS. As such, an object of the invention also includes a use of a construct for immunizing mammals, including humans, by a composition comprising antigens from multiple bacterial species, including ETEC, *C. jejuni* and *Shigella* strains. The embodied use comprises one or more priming administrations of the combination construct. The priming dose can be subsequently followed by one or more boosting doses.

### BRIEF DESCRIPTION OF THE DRAWINGS

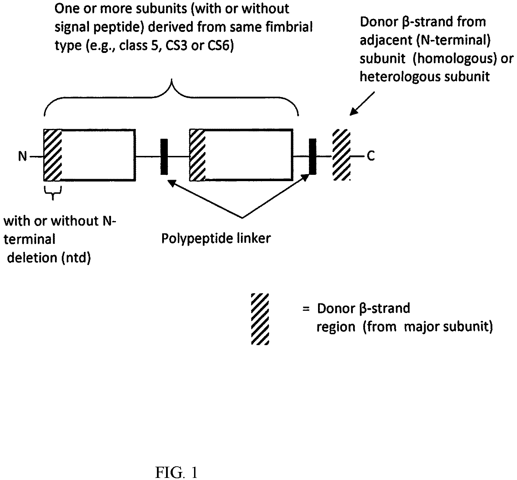

FIG. 1. Illustration of inventive construct design wherein major or minor subunits, derived from the same ETEC fimbrial type are connected, via polypeptide linkers and stabilized by donor strand complementation. The construct can contain a deletion of the N-terminal region of the N-terminal subunit. This feature prevents undesirable association with other monomers or multimers. The C-terminal subunit is stabilized by a donor β strand, connected to the subunit via a polypeptide linker, wherein the donor β strand is either derived from a homolgous subunit, which is defined as a subunit that is the same as the subunit the donor strand is stabilizing or from a heterologous subunit, defined as derived from a subunit that is different still from the same fimbrial type.

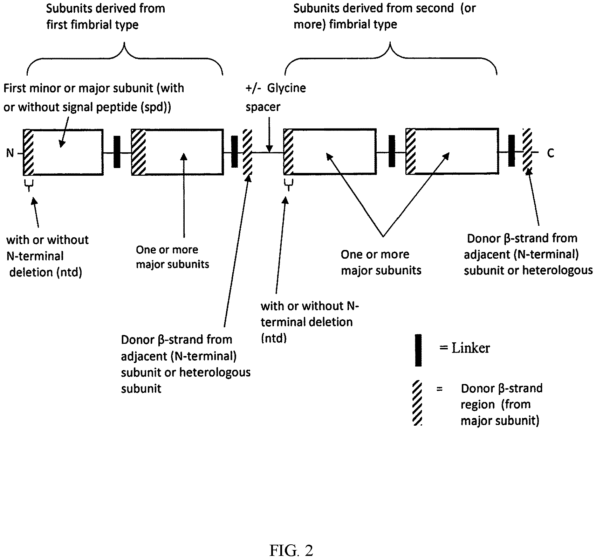

FIG. 2 illustrates a multipartite construct wherein multiple compositions, illustrated in FIG. 1, are connected via a polypeptide linker. The first subunit, is a major or minor (e.g. ETEC class 5 adhesin) ETEC fimbrial subunit. One or more major ETEC fimbrial subunits are then connected to the first subunit and to each other via a linker, wherein the subunits are stabilized by donor strand complementation. The C-terminal most ETEC major subunit is connected, via a linker, to a donor strand region from an ETEC major subunit, which can be either homologous or heterologous to the terminal major subunit. In some construct examples, in order to avoid inadvertent association of subunits, especially in CS6 subunits to each other, major ETEC fimbrial subunits can contain an N-terminal deletion of 14 to 18 amino acids.

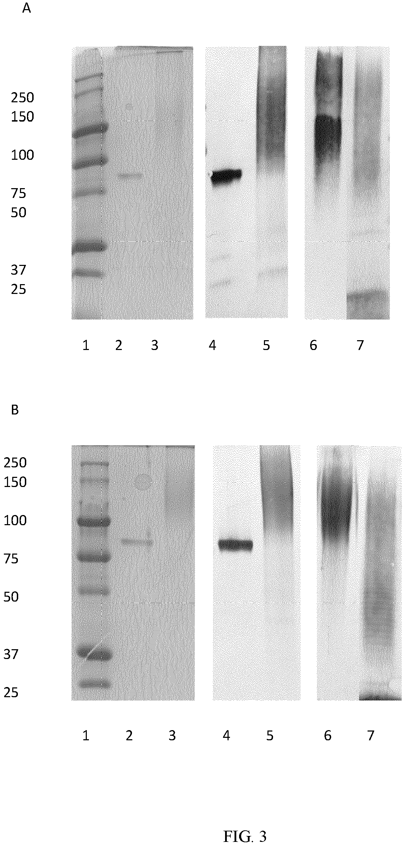

FIG. 3. SDS-PAGE and immunoblots of conjugate vaccines. A. Analyses of CfaE-HS36 conjugate. Lane 1-3 are stained with Gel Code Blue. Lane 1, Precision Plus Protein standards (BioRad); lane 2, CfaE; lane 3, CfaE-HS36 conjugate. Lanes 4-5 are immunodetected with anti-CfaE antibodies. Lane 4, CfaE; lane 5, CfaE-HS36 conjugate. Lanes 6-7 are immunodetected with antibodies to whole cells of 81-176 (HS36). Lane 6, CfaE-HS36 conjugate; lane 7, proteinase K digested whole cells of 81-176. B. Analyses of CfaEB-HS36 conjugate. Lane 1-3 are stained with Gel Code Blue. Lane 1, Precision Plus Protein standards (BioRad); lane 2, CfaEB lane 3, CfaEB-HS36 conjugate. Lanes 4-5 are immunodetected with anti-CfaE antibodies. Lane 4, CfaEB; lane 5, CfaEB-HS36 conjugate. Lanes 6-7 are immunodetected with antibodies to whole cells of 81-176 (HS36). Lane 6, CfaEB-HS36 conjugate; lane 7, proteinase K digested whole cells of 81-176. The molecular weights of the protein markers are shown on the left.
FIG. 4. *C. jejuni* anti-CPS (A) or ETEC anti-CfaE (B) induced by HS36 conjugated to CfaE or CfaEB in mice.
FIG. 5. Functional antibodies, evidenced by HAI titer, induced in mice immunized with HS36 conjugate vaccines.
FIG. 6. Summary of synthesis of polysaccharide construct and conjugation to CRM₁₉₇.
FIG. 7. Synthesis of aminopentanyl OMe-phosphoramidate galactoside. Reagent and conditions: (a) TrCl, pyridine, 95%; (b) AllBr, NaH, DMF, 0°C, 89%; (c) CAN, CH₃CN, H₂O, 0°C; then CCl₃CN, K₂CO₃, CH₂Cl₂, 57% over 2 steps; (d) HO(CH₂)₅NPhth, TMSOTf, CH₂Cl₂, 65%; (e) 80% AcOH, 80 °C, 78%; (f) PCl₂O₂Me₂, Et₃N, CH₂Cl₂, then NH₃(g), 27%; (g) PdCl₂, MeOH, 75%, (h) H₂NNH₂, EtOH, 82%.
FIG. 8. Capsule cross-reactivity to 6-MeOPN-Gal with antibodies to multiple conjugate immunogenic compositions.
FIG. 9. Serology of A. nancymaae immunized with CfaEB-HS23-36 construct. Day 0 verses day 140.
FIG. 10. HAI titers of A. nanacymaae against ETEC strain H10407 expressing Cfa 1.
FIG. 11. Immune response of mice against HS3 capsule (top panel) and against CS6 (bottom panel) following immunization with an CssBA-HS3 conjugate vaccine. The vaccine was administered at two doses, either 5 µg or 25 µg by weight.
FIG. 12. Immune response of mice to HS4 capsule (top panel) and to LTB (bottom panel) following immunization with an LTB-HS4 conjugate vaccine. The vaccine was administered at two doses, 5 ug or 25 ug by weight.

### DETAILED DESCRIPTION OF PREFERRED EMBODIMENTS

The term enterobacteria , as used herein, refers to enterotoxigenic *Escherichia coli* (ETEC), *Campylobacter jejuni* or *Shigella* spp., which include: *Shigella dysenteriae*, *Shigella flexneri, Shigella Boydii* or *Shigella sonnei.* As used herein, an enterobacteria polysaccharide polymer is a polysaccharide polymer derived from enterobacteria. The term "polysaccharide antigen" as used herein refers to a capsule polysacchride derived from *Campylobacter jejuni* (*C. jejuni* or *Campylobacter jejuni* capsule) or a lipopolysaccharide derived from *Shigella* spp.. As used herein, "polysaccharide" refers to two or more monosaccharide units composing a carbohydrate polymer molecule. A "polysaccharide polymer" refers to two or more polysaccharide molecules connected together.

The terms "polypeptide," "peptide," and "protein" as used herein can be interchangeably used, and refer to a polymer formed of two or more amino acid residues, wherein one or more amino acid residues are naturally occurring amino acids. The term "amino acid sequence" refers to the order of the amino acids within a polypeptide. As used, herein, "oligomer" are polypeptides sequences comprising relatively few amino acids.

The term "recombinant polypeptide", "recombinant polypeptide construct", or "recombinant protein", as used herein, refers to polypeptides or proteins produced by recombinant DNA techniques, i.e., produced from cells transformed by an exogenous DNA construct encoding the desired polypeptide or the desired protein. The term "recombinant construct" refers to the DNA encoding the recombinant polypeptide, recombinant polypeptide construct or recombinant protein.

The term "donor strand" or "donor β strand" refers to the N-terminal region of an ETEC fimbrial subunit that associates with another ETEC fimbrial subunit in donor strand complementation.

The term "immunogenic composition" refers to a formulation containing proteins or polypeptides or polysaccharides or polysaccharide polymers that induce a humoral and/or cellular immune response. The term "immunogenic coverage" or "spectrum of coverage" refers to the induction of humoral and/or cellular immune response against specific strains of bacteria under the "coverage." The term "immunogenic fragment" refers to a polypeptide containing one or more B- or T-cell epitopes and is of sufficient length to induce an immune response or to be recognized by T- or B-cells. The term "derivative" refers to a polypeptide or nucleic acid sequence with at least 80% identity with sequence of the identified gene. In this context, "identity" refers to two or more sequences or subsequences that are the same or have a specified percentage of amino acid residues that are the same, when aligned for maximum correspondence. Where some sequences differ in conservative substitutions, i.e., substitution of residues with identical properties, the the percent sequence identity may be adjusted upwards to correct for the conservative nature of the substitution. Percent similarity refers to proportion of identical and similar (conserved change) residues.

"Fimbriae" are defined as projections or filaments on ETEC bacteria and are composed of major subunits, as in the case of CS3 and CS6 fimbriae or major and minor subunits, as in the case of class 5a, 5b and 5c ETEC. "Fibrillae" are narrow projections from a bacteria. CS3 and CS6 fimbriae can also be termed fibrillae due to their narrow characteristic. The term "fimbrial subunit" refers to the proteins that comprise ETEC fimbriae and is used interchangeably with "pilin." "Pilin", therefore, can refer to a "major" or "minor" "fimbrial subunit" that comprise ETEC fimbriae. A "minor fimbrial subunit" refers to the adhesin protein at the tip of class 5 ETEC fimbriae and is expressed in stoichiometrically low amounts compared to "major" subunits. The "minor fimbrial subunits"include, but are not limited to, CfaE, CsfD, CsuD, CooD, CosD, CsdD, CsbD and CotD. "Major fimbrial subunits" refers to the ETEC fimbrial proteins represented in stoichiometrially larger amounts in ETEC fimbriae, compared to "minor fimbrial subunits." "Major fimbrial subunits" include the ETEC class 5 proteins: CfaB, CsfA, CsuA2, CsuA1, CooA, CosA, CsdA, CsbA, CotA; the ETEC CS3 proteins: CstH, CstG; and the ETEC CS6 proteins: CssA, and CssB.

The pathogenesis of *Campylobacter jejuni* remains poorly understood in comparison with ETEC and the organism shares few virulence factors with better-characterized pathogens. *C. jejuni* is unusual, however, among enteric pathogens in that it expresses a polysaccharide capsule (CPS) that is one of its few confirmed virulence factors.

Because of the importance of ETEC and *C. jejuni* as pathogenic agents, a combined ETEC-CJ composition was constructed in order to afford protection against both agents. In one embodiment, a recombinant polypeptide construct, comprising fimbrial subunits from Class 5 ETEC strains is fused to a capsule polysaccharided from the *C. jejuni* strain 18-176.

In a preferred embodiment, one or more recombinant polypeptide ETEC constructs, comprising the ETEC fimbrial adhesion, are conjugated to isolated *C. jejuni* capsule polysaccharide (CPS). One or more of a number of ETEC recombinant constructs can be conjugated to one or more of a number of C. jejuni capsule polysaccharide structures. In the inventive construct, the ETEC recombinant construct operates both as an immunogen against ETEC and as a protein carrier molecule, presenting the *C. jejuni* polysaccharide. Examples of ETEC recombinant polypeptides and *C. jejuni* capsule polysaccharides that can be incorporated into a combined structure are given in the following examples.

In a preferred embodiment, the ETEC polypeptide construct can not only serve as antigen against ETEC but also serve as a protein carrier for polysaccharide antigens, such as *C. jejuni* capsule polysaccharide.

### Example 1: Conjugation of ETEC polypeptides to C. jejuni capsule polysaccharide (CPS)

In a preferred embodiment, ETEC recombinant polypeptides or polypeptide constructs are conjugated to *C. jejuni* CPS. The CPS can be derived from a number of *C*. *jejuni* strains. In the embodiment, any CPS of any *C. jejuni* strain is envisioned to be conjugated to ETEC recombinant polypeptide constructs. Alternatively, *Shigella* LPS can be conjugated to ETEC recombinant polypeptide constructs.

The overall method of conjugating includes oxidizing *C. jejuni* CPS, for example, with NaIO₄ in sodium acetate (pH 4.0). Oxidized CPSs were desalted with a 5 kDa cutoff membrane by stirred ultrafiltration, which is subsequently lypholized. ETEC proteins are then added. The stoichiometery protein to CPS can vary, however, a typical ratio is 1:2 protein to CPS by mass. The concentration of components can be by any method. However, for example, polysaccharide concentration was determined by antrhone assay and protein concentration was determined. NaCNBH₃ is then added. The conjugates are subsequently desalted by ultrafiltration and lyophilized. CPS (or *Shigella* LPS), ETEC proteins and conjugates were analyzed, for example by SEC-HPLC. Conjugates were also analyzed by SDS polyacrylaminde gel electrophoresis (PAGE) and Gel Code ^{®} Blue (Pierce, Biotechnology, Inc, Lombard, IL) staining. Conjugates were detected by antibody-based assay using anti-CPS and anti-ETEC protein.

As an example of ETEC recombinant polypeptide and *C. jejuni* conjugation, the CPS from the *C. jejuni* strain 81-176 was conjugated to ETEC recombinant polypeptide construct CfaE (class 5 ETEC adhesin) or to the recombinant polypeptide construct CfaE linked, via a polypeptide linker, to the major subunit CfaB.

CPS *C. jejuni* capsule was purified from *Campylobacter jejuni* strain 81-176 (PG3208). This mutant, in which the *gal*T gene was insertionally inactivated by chloraphenicol cassette, lacks all ganglioside mimicry in its lipoligosaccharide (LOS) core.

The cells were grown in porcine Brain-Heart Infusion (BHI) broth and sonicated to inactivate the cells. The CPS was extracted by hot water/phenol method previously employed for the same organism (Chen, et al., Carbohyd. Res. 243: 1034 (2008)). Cells were immersed in a water/phenol mixture (3:2 ratio by volume), which was heated to 67°C with stirring for 4 hours. The suspension was cooled and separation of the mixture into two separate layers (the aqueous layer and the phenol layer) and extraction of the aqueous layer was performed. The aqueous layer was removed and the phenol/water extraction was repeated on residue, to maximize the yield. Aqueous layers from two extractions were pooled and boiled fro 1.5 hours with the aditon of acetic acid to a pH of 3.5. The aqueous layer was dialyzed gainst running water for 2 days and concentrated using a Millipore concentrator cell with a 5 kDa cutoff membrane. Trace amounts of residual RNA were removed by digestion with benzonase enzyme at 90 u/ml in 50 mM Tris-CCl/1 mM MgCl₂, pH 8 overnight at 37°C. Benzonase was removed from CPS, then desalted and concentrated using stirred ultrafiltration with 30 and 5 kDa MWCO disc membranes, respectively.

The isolated CPS was oxidized with adding 40 mg of CPS to 40 mM NaI0₄ in sodium acetate pH 4 in the dark at 4°C for 2 days. Oxidized CPS was desalted with 5kDa cutoff membrane by stirred ultrafiltration and was subsequently lyophilized.

Prior to conjugation the ETEC proteins, for example dscCfaE and dscCfaEB, were transferred to 0.1M borate buffer at pH 9.0. Oxidized CPS was added to each ETEC protein at a ratio of 1:2, protein to CPS by mass, and then NaCNBH₃ was added at 2 times mass equivalent to CPS. The reaction was incubated 1 day at room temperature and 6 days at 37°C in the dark with continuous stirring. The conjugates were desalted by stirred ultirafiltration with 30 kDa membrane and lyophilized. Conjugates of the CPS to dscCfaE and dscCfaEB was conducted by SEC-HPLC. and polyacrylamide gel (PAGE) (12.5%) electrophoresis.

In PAGE analysis, immunodetected with rabbit polyclonal antibodies to whole cells of 81-176 was used to detect CPS and to CfaE. The results of this study are shown in FIG. 3. Immunoblotting of both conjugates with anti-CfaE antisera confirmed that the proteins ran as high molecular weight conjugates with conjugates with apparent masses ranging from just higher than the mass of each respective protein to >250 kDa. Immunoblotting with antisera to formalin fixed whole cells of C. jejuni 81-176 confirms that capsular polysaccharide was conjugated to the proteins. As illustrated in FIG. 3, no unconjugated protein remained in either conjugation.

The results of FIG. 3 were confirmed in SEC-HPLC. In the SEC-HPLC, unoxidized and oxidized CPSs, ETEC proteins and conjugates were analyzed using SEC-HPLC with a TSKgel-G2000SWₓ₁ column (30 cm x 7.8 mm ID) and TSKgel SW guard column run on an ICS-5000 Dionex system with 0.1 M phosphate at pH 6.8, 0.1 M sodium sulfate and 5% acetonitrile at 0.6 ml/min flow rate. Samples were monitored at 214 nm with Ultimate 3000 variable wavelength detector and RI detector, both from Dionex.

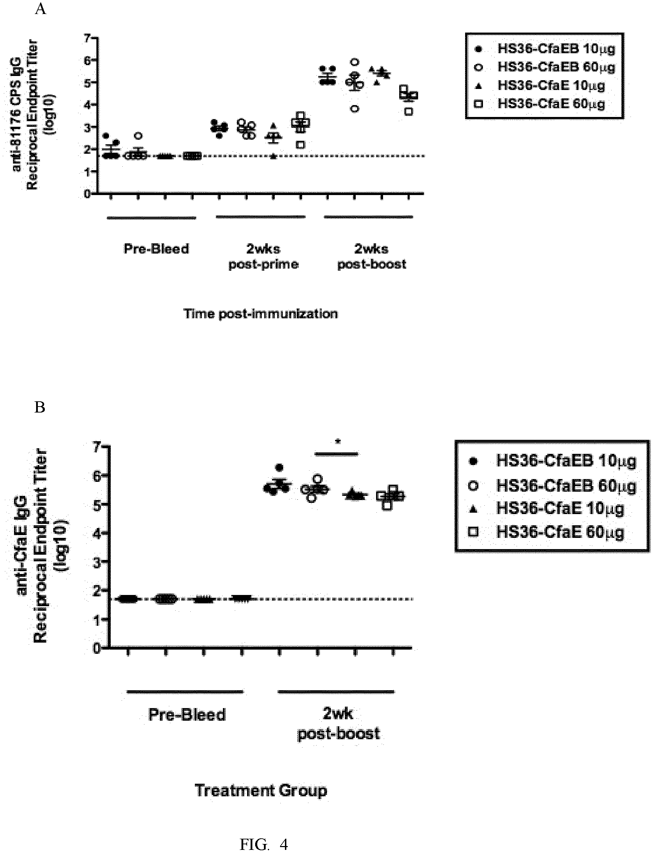

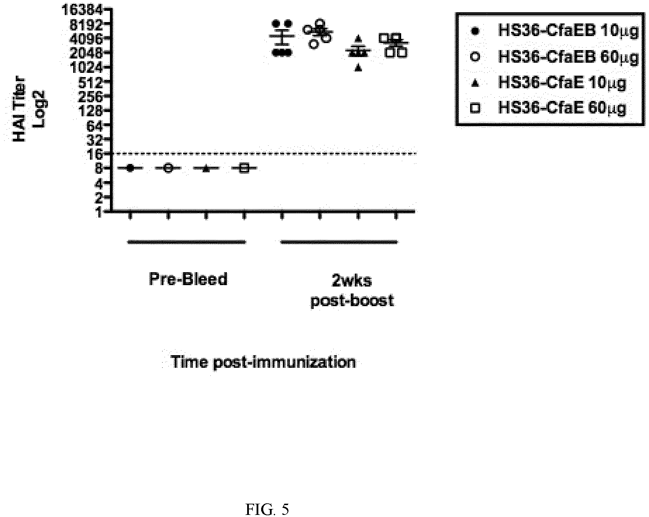

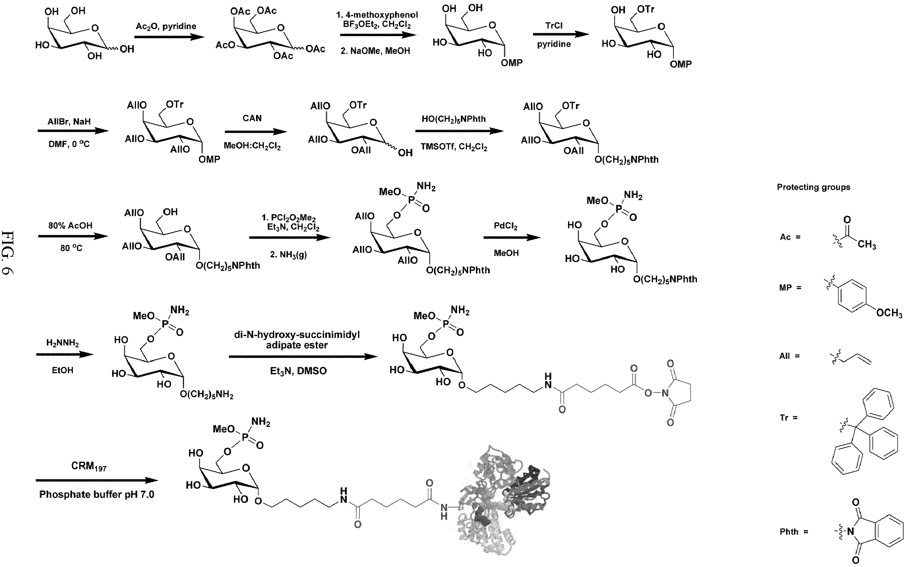

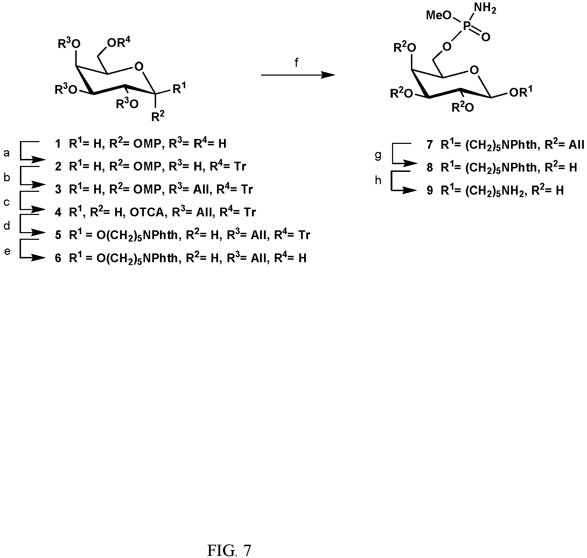

The results of the SEC-HPLC are shown in FIG. 4 for dscCfaEB and in FIG. 5 for dscCfaE. Analysis by matrix-assisted laser desorption/ionization (MALDI) is shown in FIG. 6, for dscCfaEB and FIG. 7 for CfaE.

Detection of the conjugates by refractive index (RI) on SEC-HPLC revealed that 45% and 50% of the polysaccharide remained unconjugated with the CfaE and CfaEB conjugates respectively. This is summarized in Table 1, which also illustrates that the conjugated molar ratio of CPS to CfaE was 4.8:1 and that of CPS to CfaEB was 4.4:1.

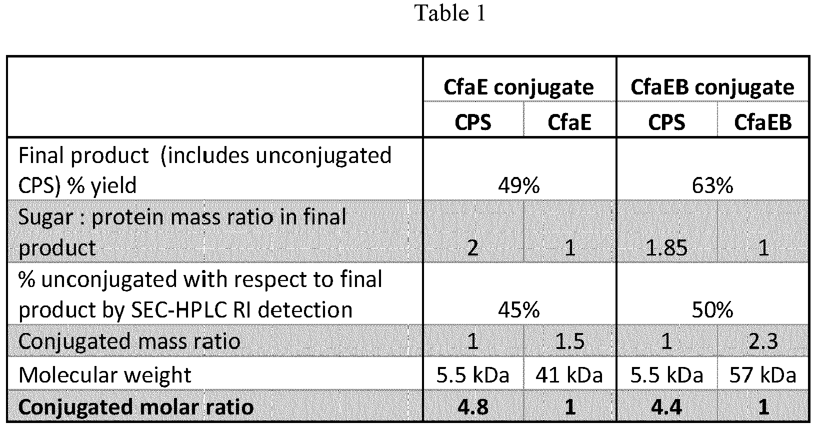

### Example 2: Anti-Class 5 ETEC, CS3 or CS6 constructs

Anti-ETEC constructs that are contemplated to be conjugated to *C. jejuni* polysaccharide comprise the structures as illustrated in FIG. 1 and FIG. 2. FIG. 1 illustrates the basic recombinant construct design. As diagrammed in FIG. 1 the construct design comprises one, or more ETEC major or minor fimbrial subunits or fragments of major fimbrial subunits, containing the donor strand, derived from the same ETEC fimbrial type, which are connected, via polypeptide linkers and stabilized by donor strand complementation. The construct can contain a deletion of the N-terminal region of the N-terminal subunit. This feature prevents undesirable associations with other monomers or multimers. The C-terminal subunit is connected to and stabilized by a donor β strand, connected to the subunit via a polypeptide linker, wherein the donor β strand is either derived from the adjacent subunit (i.e., homologous) or from a different subunit of the same fimbrial type (i.e., heterologous).

FIG. 2 illustrates the basic multipartite construct, wherein multiple constructs as in FIG. 1, are connected forming a recombinant construct comprising two or more fimbrial types. As illustrated in FIG. 1, major or minor subunits from the same fimbrial type are connected via a polypeptide linker sequence. In the multipartite construct, two or more constructs, as in FIG. 1, are connected, via a linker polypeptide.

In the multipartitie construct design, as in the basic design (compare FIG. 1 with FIG. 2), the first subunit (N-terminal) is a major or minor ETEC fimbrial subunit. Each additional subunit is connected to adjacent subunits via a polypeptide linker that enables rotary freedom of the molecular components. The subunits are associated with and stabilized via a donor strand complementation from a C-terminally adjacent subunit via a donor β strand, connected via a linker polypeptide, to the C-terminus of the stabilized subunit. In some embodiments, subunits can contain a deletion of 14 to 18 amino acids from its N-terminal end. Additionally, specific constructs can be constructed with or without signal peptides of 18 to 22 amino acids and with or without histidine tags at the C-terminus.

In the multipartite construct, subunits from the same fimbrial type are directly connected. Groupings of subunits from the same fimbrial type are then connected to other groupings of subunits from other fimbrial types. Fimbrial types include, but are not limited to ETEC class 5a, 5b, 5c, CS3 and CS6. For example a single construct can include subunits derived from any two or more of class 5a, 5b, 5c, CS3 and CS6 fimbrial types.

Multiple linker sequences can be utilized in connecting the individual subunits. Examples of specific linkers include the tetrapeptide of SEQ ID No. 5. Another example is a tri-glycine linker (i.e., G-G-G). In the inventive construct, i*n cis* donor strand complementation is used to stabilize adhesins and adhesin-pilin fusions for representative Class 5a, 5b, and 5c adhesins.

The contemplated composition is designed to enable as wide a range of coverage of ETEC strains as possible. As such, in one embodiment, the contemplated composition and use is aimed at inducing immunogenic response against class 5a, 5b, 5c ETEC, as well as ETEC strains expressing CS3 or CS6 fimbrial components.

In a preferred embodiment, recombinant polypeptide ETEC constructs are conjugated to *C. jejuni* capsule polysaccharide (CPS). One or more of a number of ETEC recombinant constructs can be conjugated to one or more of a number of C. jejuni capsule polysaccharide structures. Examples of Class 5 ETEC recombinant polypeptides are listed in Table 2. In Table 2, minor subunits are stabilized by connection, via a polypeptide linker, to associated major subunits. Alternatively, a 12-16 amino acid donor strand, derived from the associated major subunit is connected to the minor subunit via a polypeptide linker. These polypeptides can also be linked as per FIG. 1 and FIG. 2 to lead to the example constructs listed in Table 3. These examples can then be conjugated to isolated *C. jejuni* capsule polysaccharide, as in Example 1.

**Table 2**

| Immune coverage (fimbrial types) | Subunit | | SEQ ID No. Full length sequences including spd¹ (DNA/polypeptide)) | SEQ ID No. Mature sequences (DNA/polypeptide)² |
|---|---|---|---|---|
| Class 5a | CfaE | | 56/57 | 115/58 |
| | CfaB | | 59/60 | 116/61 |
| | CsfD | | 64/65 | 117/88 |
| | CsfA | | 62/63 | 118/89 |
| | CsuD | | 70/71 | 119/90 |
| | CsuA2 | | 68/69 | 120/91 |
| | CsuA1 | | 66/67 | 121/92 |
| Class 5b | CooD | | 74/75 | 122/93 |
| | CooA | | 72/73 | 123/94 |
| | CsdD | | 78/79 | 124/95 |
| | CsdA | | 76/77 | 125/96 |
| | Cos D | | 82/83 | 133/97 |
| | CosA | | 80/81 | 126/98 |
| | CsbD | | 44/45 | 127/46 |
| | CsbA | | 47/48 | 128/49 |
| Class 5c | CotD | | 50/51 | 129/52 |
| | CotA | | 53/54 | 130/55 |
| CS3 | CstH | | 84/85 | 131/99 |
| | CstG | | 86/87 | 132/101 |
| CS6 | CssA | | 134/135 | 1/2 |
| | CssB | | 136/137 | 3/4 |

| | | | | |
|---|---|---|---|---|
| ¹ "spd" refers to signal peptide. The mature polypeptide sequence, therefore, would be the full length minus the signal peptide. ²DNA sequence encodes mature protein. | | | | |

**Table 3**

| **Fimbriae class represented** | **Construct (adhesin-pilin) example¹** | **SEQ ID No. (Protein/DNA)³** |
|---|---|---|
| Class 5a | dsc_{14CsfA}CfaE-CfaB-CsuA2-CsfA. | 103/104 |
| Class 5b | dsc_{14CsbA}CsbD-CsbA-ntd₁₅dsc_{14CooA}CooA² | 105/106 |
| Class 5b | dsc_{15CooA}CsbD-CsbA-CooA² | 107/108 |
| Class 5c | dsc_{14cotA}CotD-CotA | 109/110 |

| | | |
|---|---|---|
| ¹dsc refers to donor strand complementation. The number and subunit refers to the N-terminal amino acids of length represented by the number from the subunit indicated that is connected at the C-terminus of the construct and is serving to stabilize the C-terminal construct. For example, "dsc_{14CsfA}" refers to the N-terminal 14 amino acids of CsfA connect to the C-terminus of the construct. ²Linkers polypeptides are GGG rather than DNKQ. ³Sequence in example contains a Leu-Glu-His₆ at the C-terminus. | | |

An important feature of the anti-ETEC construct is the enhanced immune recognition of the fimbrial adhesion. The minor subunits (i.e., ETEC adhesin) of ETEC Class 5 fimbriae are stoichiometrically represented in very low numbers relative to the major subunit. Therefore, an important feature of the recombinant constructs is the vastly improved stoichiometric representation of the minor subunit in order to enhance immune recognition of the minor subunit. Additionally, since fimbrial subunits, such as CfaE, are relatively susceptible to proteolytic degradation outside of the fimbrial structure, stabilization of the adhesin is also important. Therefore, constructs are designed to express ETEC subunits stabilized from misfolding and degradation by donor strand complementation.

The donor β strand is provided by the major fimbrial subunit. For example, in the case of CfaE, stabilization is provided by the N-terminal region of CfaB. Engineering of dscCfaE by incorporation of a donor peptide strand from the N-terminus of the CFA/I major subunit CfaB at its C-terminus transformed an insoluble, unwieldy native, recombinant protein into a stable immunogenic composition (Savarino, U.S. Patent application publication no. 20060153878 (13 July 2006)), which is incorporated by reference, herein.

Based on its atomic structure, dscCfaE is folded into a native, β-sandwich conformation, consisting of two half-barrels, comprising the N-terminal adhesin domain (CfaEad) a short α-helical connector, and the C-terminal pilin domain (CfaEpd). The molecule is functional in that it directly mediates MRHA of bovine and human erythrocytes, and generates neutralizing antibodies that act to inhibit MRHA and decorate the tips of CFA/I fimbriae on immunoelectron microscopy.

A fusion protein was engineered by genetic insertion of the coding sequence for mature major structural subunit of ETEC adhesin, such as CfaB, to the 3'-end of the minor subunit, such as CfaE. This concept was disclosed in Savarino, U.S. Patent application (11/340,003, filed January 10, 2006), which is incorporated, herein. This molecule contains all three domains of the CFA/I fimbriae (i.e., ad, pd, and major subunit) in a ratio of 1:1:1, rather than that found in native fimbriae (ca. 1:1:1000).

A number of observations indicate the suitability of dscCfaE (cloned from ETEC strain E7473) as a vaccine antigen. First, sequencing of 31 different wild type alleles of *cfaE* from ETEC isolates of varying geographic origin and serotypes, show that the gene and predicted polypeptide sequence are nearly invariant, with three different nonsynonymous nucleotide changes at one site each in only five of these 31 alleles (Chattopadhyay, etal., J. Biol. Chem., 287(9): 6150-6158 (2012)). Hence, the target protein shows uniformity in natural ETEC bacterial populations.

Additionally, CfaE, a Class 5a fimbrial adhesin, is 80-81% identical with the other Class 5a minor subunits proteins adhesins CsuD of CS14 fimbriae and CsfD of CS4 fimbriae. CsuD and CsfD share 94% identity. This is considerably higher than the average identity with other Class 5b and 5c fimbrial adhesins (mean 50% identity).

Moreover, rabbit anti-dscCfaE serum cross-neutralizes CS4- and CS14-ETEC in the hemagglutination assay (HAI). A number of vaccination studies have been performed in small (rabbit and mice) and large (monkeys and cows) animals with various routes of administration and adjuvant combinations showing that dscCfaE is a potent immunogen that can elicit systemic and mucosal antibodies which recognize dscCfaE and CFA/I and are neutralizing (as measured by HAI assay).

An embodiment includes anti-class 5 ETEC constructs based on the construct design illustrated in FIG. 1, whereby the N-terminal subunit is an ETEC class 5 minor (i.e., adhesin) subunit, listed in Table 2, including CfaE, CsfD, CsuD, CooD, CsdD, CosD, CsbD and CotD, connected, via a polypeptide linker, to one or more ETEC major subunits, from the same ETEC class 5 type, listed in Table 2. The polypeptide linker can be any of a number of polypeptide sizes. In a preferred embodiment, the linker is a tetrapeptide with the polypeptide sequence of SEQ ID No. 5. The C-terminal class 5 subunit is connected to a donor β strand, derived from a homologous subunit and is typically 12-19 amino acids. In alternative embodiments, one or more major subunit can include a deletion of 12 to 16 amino acids from the N-terminal region of the subunit.

The design in FIG. 1, utilizes the concepts disclosed in Savarino, U.S. Patent application (11/340,003, filed January 10, 2006)), including donor strand complementation to provide stabilized class 5 ETEC adhesin. Due to the homology of ETEC class 5 minor subunits and major subunits, FIG. 1 further contemplates multiple constructs incorporating the fimbrial subunits of Table 2, or derivatives of these polypeptides or DNA sequences.

The construct design, illustrated in FIG. 1, incorporates the donor strand complementation stabilization features of Savarino (U.S. Patent application (11/340,003, filed January 10, 2006)), and furthers it by incorporating multiple major subunits, from a specific ETEC type, into a single adhesin-pilin construct. For example, multiple class 5b major subunits can be connected to a class 5b adhesin (i.e, minor subunit). Embodiments include adhesin-pilin constructs containing Csb D (ETEC Class 5b fimbrial adhesin) and Cot D (ETEC Class 5c fimbrial adhesin). Examples, for illustration, of embodiments of adhesin-pilin ETEC class 5 adhesin-pilin constructs, representing Class 5a, 5b and 5c are shown in Table 3.

### CS6 and CS3

Rabbit model (RITARD) studies suggest the colonization factor CS6 and CS3 has immune-protective potential (Svennerholm, et al., Infect. Immun. 56: 523-528 (1988); Svennerholm, et al., Infect. Immun. 58: 341-346 (1990)). As such, an important technical goal is to reproduce a stabilized CS6 expressing recombinant structure expressing CS6 antigens that maximally elicits antibody responses inhibitory to CS6-directed adhesion.

Unlike class 5 ETEC fimbriae, the fimbrial structures may function as polyadhesins rather than monadhesins (Zavialov, et al., FEMS Microbiol. Rev. 31: 478-514 (2007)). Extrapolation from related fimbriae, assembly of ETEC CS6 and CS3 may be mediated by a donor strand complementation mediated process through association of a CS6 or CS3 subunit with the N-terminal donor strand region of an adjacent subunit. Additionally, protection against misfolding and proteolytic degradation may also be afforded through donor strand complementation.

Association of monomers of CS3 and CS6 was evaluated by visualization of the subunit proteins under denaturing and non-denaturing conditions in polyacrylamide gel electrophoresis (PAGE). For both CS3 and CS6 monomers, under denaturing conditions the proteins migrating at the expected sizes. Under non-denaturing conditions multiple size (i.e., ladders) are seen formed by multimeric association of the subunits.

### CS6 fimbriae

CS6 fimbriae comprise CssA and CssB. Whereas the two CS3 major subunits show little to no variation in polypeptide sequences, modest variation in CS6 proteins is observed. For example, greater than 90% identity is found in CS6 protein CssA and greater than 95% identity is found in CssB allotypes. Both CS6 structural proteins exhibit a relatively low level of variation (i.e., greather than 90% amino acid conservation), with greater variation in CssA and the mutations randomly distributed along the CssA polypeptide.

In order to design an effective immunogenic composition that would be suitable for inclusion in a vaccine formulation a number of criteria were devised for determination of suitable constructs. These included the ability to maintain a structure without unwanted self-association or assembly; thermostability; and ability to generate anti-CS6 IgG and IgA antibody levels similar to those elicited by immunization with CS6.

Monomeric CS6 subunit assembly appears to be mediated by donor strands from adjacent CS6 subunits, as discussed above. It is hypothesized that interaction to form these stable structures is mediated by inter-subunit interaction through donor strand complementation. Donor strand complementation also affords protection against misfolding and proteolytic degradation. Therefore, in a preferred embodiment, multimeric CS6 constructs were developed to take advantage of these attributes of donor strand complementation. Additionally, multimeric expression provides more efficient manufacture over production of monomers.

In one embodiment, a construct conjugated to *C. jejuni* comprises a multimeric CS6 with one or more of the CS6 subunits, CssA and CssB, or allelic variation or derivatives, with the construct design configuration illustrated in FIG. 1. In a preferred embodiment, the construct comprises a dimer of CssB and CssA with CssB N-terminal to CssA (i.e., CssB-CssA).

As illustrated in FIG. 1, or FIG. 2, CS6 subunit association is stabilized by *in cis* donor β strand complementation. Donor strand complementation is afforded by linking a CS6 subunit at its C-terminus, to the donor β strand region of another CS6 subunit, via a tetrapeptide linker. The linker can be any of a number of polypeptide regions. However, in a preferred embodiment, the linker is either as in SEQ ID No. 5 or a triglyicine linker. In the case of a terminal CS6 subunit, stabilization is provided by donor β strand, connected at its C-terminus, from a homologous or heterologous CS6 subunit. Homologous subunit is defined as two subunits of the same form (e.g., CssA OR CssB). Heteterologous subunits are of different forms (e.g., one is derived from CssA the other from CssB. The CS6 donor β strand is typically the N-terminal 14-16 amino acid region of CS6 subunit. The recombinant protein can be constructed with or without hexahistidine affinity tags, which are typically on the C-terminus.

Additionally, to prevent recombinant polypeptide constructs forming molecular associations resulting in un-desirable non-covalent oligomer formation, in a preferred embodiment, the N-terminal 14-16 amino acids of the N-terminal CS6 subunit is deleted. As an illustration, "dsc_{B14}CssBA" would contain a heterologous donor strand (i.e., "dsc"), from CS6 CssB, inserted at the C-terminus of the construct. In this case, the donor strand is 14 amino acids in length, as indicated by the "14." Similarly, a constructed designated "ntd₁₅dsc_{A}CssBA" would contain a homologous donor strand at the C-terminus of the construct and also comprises a deletion of the N-terminal amino acid region (termed "ntd").

Examples of constructs comprise one or more CS6 subunits with amino acid sequences sequences selected from the group consisting of SEQ ID No. 2 (CssA) or SEQ ID No. 4 (CssB), or derivatives of these polypeptides. The DNA sequence for CssA is SEQ ID No. 1 and for CssB, SEQ ID No. 3. The subunits are connected by a polypeptide linker sequences. In a preferred embodiment, the linker is a tetrapeptide with the amino acid sequence of SEQ ID No. 5.

### CS3 fimbriae

Savarino (U.S. Patent application No. 11/340,003 (2006)) discloses donor strand complementation stabilized ETEC constructs. Embodiments of this application incorporate the donor strand stabilization of CstH and adds the second CS3 subunit, CstG. Embodiments herein add additional features found to be important for stabilization of the CS3 subunits and immunogenicity against CS3. CS3 comprises CstH and CstG. The CS3 structural protein CstH is invariant. CstG is also highly conserved, showing 99-100% identity in polypeptide sequence for 39 wildtype CS3 genes sequenced. Similiarly, although some variation CstG is observed, it is also relatively invariant, with 99-100% amino acid conservation.

CS3 contains both CstG and CstH, in near equal amounts. Therefore, dimeric constructs were devised incorporating CstG and CstH, according to the template construct design of FIG. 1.

In one embodiment a polypeptide construct conjugated to C. jejuni capsule polysaccharide comprises a CS3 s construct designed according to FIG. 1. In FIG. 1, CS3 constructs comprise one or more CS3 fimbrial subunits connected via a polypeptide linker. The C-terminal fimbrial subunit is connected, via a polypeptide linker, to a donor β strand region of a CS3 fimbrial subunit. The C-terminal donor β strand can be derived from the same CS3 subunit to which it is connect (i.e., homologous) or derived from a different subunit (i.e., heterologous). The polypeptide linker can be any number of polypeptide regions, however, in a preferred embodiment, the linker is a tetrapeptide of the sequence of SEQ ID No. 5, or a triglycine (i.e., G-G-G). The donor β strand region is the N-terminal 14 - 16 amino acids of the mature CstH or CstG protein. In alternatives of this embodiment, the first 14 - 18 amino acids of the N-terminal region of the N-terminal most subunit is deleted to avoid undesirable associations.

In a preferred embodiment, the CS3 construct is a dimer. Although other examples are contemplated using the design of FIG. 1, as an illustrative example, the recombinant polypeptide construct can be configured as "dsc_{16CstH}CstG-(linker)-CstH". In this example, the mature CstG polypeptide (SEQ ID No. 101) or full length polypeptide sequence (SEQ ID No. 87) is connected at its C-terminus to CstH polypeptide (SEQ ID No. 99), via a polypeptide linker. In this example, the CstH polypeptide, is connected, at its C-terminus, to a donor β strand region of 16 amino acids derived from CstH via a polypeptide linker.

Other examples can include constructs, according to FIG. 1. In other examples, the C-terminal donor β strand can be either homologous (derived from the same subunit) or heterologous (derived from a different subunit) to the C-terminal most CS3 fimbrial subunit.

### Construction of multipartite fusion constructs

Immunity to multiple strains of ETEC is important to obtain the greatest extent of anti-ETEC immunity. Toward this goal, recombinant polypeptide constructs were developed comprising two or more subunits derived from different ETEC fimbrial types according to the design illustrated in FIG. 2 to form multipartite fusion constructs. As used, herein, multipartite fusion or multipartite fusion constructs are recombinant polypeptide constructs according to FIG. 2. In this design, different ETEC fimbrial types are defined as fimbrial proteins derived from fimbriae of different strain ETEC types, as listed in Table 4 or 5, or deriviates of these polypeptides or DNA sequences. For example, the fimbrial type "CS3" comprises CstH and CstG. The fimbrial type "CS6" comprises CssA and CssB. The fimbrial types of Class 5 ETEC include the fimbrial types Class 5a, Class 5b and Class 5c.

In a preferred embodiment, major and/or minor subunits, derived from the same ETEC fimbrial type are connected, via polypeptide linkers, and stabilized by donor β strand complementation, as illustrated in FIG. 1. A multipartite fusion comprises one or more fimbrial subunits of the same fimbrial type, as in FIG. 1, connected to one or more fimbrial subunits derived from a different fimbrial type as illustrated in FIG. 2.

In one embodiment, the multipartite fusion construct can include a deletion of the N-terminal region of one or more fimbrial subunits, but is preferably on the N-terminal most fimbrial subunit for a given ETEC fimbrial type, as illustrated in FIG. 2. This feature prevents undesirable associations with other monomers or multimers. The size of the deletion of the N-terminal region is 14 to 18 amino acids. In other embodiments, multipartite fusion constructs comprising Class 5 adhesins do not contain a deletion of the N-terminal region.

As illustrated in FIG. 2, the C-terminal subunit, for an ETEC fimbrial type, is connected to and stabilized by a donor β strand, connected to the subunit via a polypeptide linker, wherein the donor β strand is either that derived from the adjacent subunit (i.e., homologous) or from a different subunit of the same fimbrial type (i.e., heterologous). The size of the N-terminal donor strand depends on the fimbrial type and subunit stabilized. In preferred embodiments, for class 5 fimbrial subunits, the donor β strand, derived from the N-terminal region of the class 5 subunit stabilized, is 12 to 16 amino acids. For CS3 and CS6 subunits, the donor β strand is 14 to 16 amino acids. As mentioned above, the construct can contain a deletion of the N-terminal region of the N-terminal subunit. This feature prevents undesirable associations with other monomers or multimers. The size of the deletion of the N-terminal region is 14 to 18 amino acids.

As illustrated in FIG. 2 multiple constructs as in FIG. 1 are connected forming a recombinant polypeptide construct comprising two or more ETEC fimbrial types. In this way, one or more major or minor subunits, derived from the same ETEC fimbrial type, are connected via polypeptide linkers and stabilized by donor strand complementation. In another embodiment, one or more glycine residues separates different ETEC fimbrial types, acting as a "swivel" means between the ETEC types. The glycine residue, due to its small, unbranched molecular characteristics, enables rotary freedom of the molecular components. Subunits derived from the same fimbrial type (as in FIG. 1) are connected by a polypeptide linker, with the subunits stabilized by donor strand complementation. As shown in FIG. 2, the C-terminal subunit of each ETEC fimbrial type is stabilized by a donor β strand that is homologous or heterologous to the C-terminal subunit of that fimbrial type.

In other embodiments, the construct can contain an N-terminal deletion at the N-terminus of the entire construct as well as an additional deletion, of 14 to 18 amino acids, at the N-terminus of the first "internal" subunit that is of a different fimbrial type. This is illustrated in FIG. 2. In the case of the deletion on the N-terminus of the "internal" subunit, the deletion serves to shorten the length between subunits, thus reducing the likelihood of misfolding and proteolytic cleavage. In another embodiment, a donor β strand, derived from a homologous or heterologous subunit, is inserted at the C-terminus of the C-terminal CS6 or CS3 subunit. For class 5 fimbrial subunits, the donor β strand, derived from the N-terminal region of the class 5 subunit that is stabilized, is 12 to 16 amino acids. For example, in preferred embodiments, CfaB is stabilized by a 14 amino acid donor β strand; CsfA by a 14 amino acid donor β strand; CsbA by a 15 amino acid donor β strand, CooA by a 14 amino acid donor β strand and CotA by a 14 amino acid donor β strand. For CS3 and CS6 subunits, the donor β strand is 14 to 16 amino acids, with preferred embodiments of CS3 fimbrial subunits (i.e., CstH or CstG) stabilized by a 16 amino acid donor β strand derived from CstH or CstG; and CS6 fimbrial subunits (i.e., CssA or CssB) stabilized with a 16 amino acid donor β strand derived from CssA or CssB. However, other donor β strand lengths are envisioned.

The inventive compositions can utilize different linker sequences. In a preferred embodiment, the linker contains the amino acid sequence of SEQ ID No. 5. In another embodiment, the linker is a tri-glycine linker. In other embodiments, the C-terminal end of the construct contains a histidine tag for purification of the construct.

In the inventive construct, *in cis* donor strand complementation is used to stabilize adhesins and adhesin-pilin fusions for representative Class 5a, 5b, and 5c adhesins. For each adhesin target group, in a preferred embodiment, the compositions are constructed with the intent of eliciting anti-adhesive immune responses. Further towards this goal, Class 5 multipartite fusions comprising Class 5 adhesin minor subunits are typically construct such that the adhesin (i.e., minor fimbrial subunit) is located at the N-terminus of the constructed with the minor fimbrial subunit linked at its C-terminus to one or more major subunits, followed at the terminal end of the construct with the donor β-strand of the last major subunit.

Other embodiments include constructs comprising Class 5a adhesin CfaE tandemly linked at its C-terminus to one or more of CfaB (CFA/I major subunit), CsuA2 (CS14 major subunit) and CsfA (CS4 major subunit); Class 5b adhesin CsbD tandemly linked at its C-terminus to one or more of CsbA (CS17 major subunit), which shares high identity to the CS19 pilin subunit CsdA, and CooA (CS1 major subunit), which shares high identity to the PCFO71 pilin subunit CosA; and Class 5c adhesin CotD tandemly linked at its C-terminus to CotA (CS2 major subunit).

Embodiments of ETEC multipartite fusion constructs are illustrated in Table 4 and 5. In this embodiment, constructs comprise any major or minor ETEC fimbrial subunit from Table 2 in multiple combinations, connected by linker polypeptides and stabilized from proteolytic degradation by donor strand complementation utilizing the design illustrated in FIG. 2. Table 2 lists the ETEC fimbrial subunits (major and minor subunits) than can be used and incorporated into the multipartite fusion construct design of FIG. 2, which can then be conjugated to *C. jejuni* capsule polysaccharide or *Shigella* LPS. Any subunit, therefore, is combined with one or more other ETEC major subunits from any ETEC fimbrial phenotypic type, including Class 5a, 5b, 5c, CS3 and CS6.

The recombinant polypeptide construct motif comprises a whole or immunogenic fragment of a minor or major ETEC fimbrial subunit connected at its C-terminal end to a linker. The linker is connected at its C-terminus to a whole major ETEC fimbrial subunit or a polypeptide donor strand of an ETEC major structural subunit, derived from the same fimbrial type. The whole ETEC major subunit or donor strand polypeptide is then connected, via a linker at its C-terminal end, to one or more additional major structural fimbrial subunits, derived from the same fimbrial type, from Table 2.

The strategy for selecting and developing specific genetic fusion constructs is guided, in part, by the phylogenetic and antigenic relatedness of subunits. For example, constructs containing Class 5a, 5b and 5c pilin subunits are selected based on the relatedness of minor and major subunits within a particular ETEC fimbrial class (i.e., class 5a, 5b or 5c). As such, adhesin (i.e., minor fimbrial subunit) from a specific fimbrial type (e.g., Class 5a) are linked to Class 5a major subunits. Further selection of subunits is guided and based on epidemiological study analysis in order to achieve optimum immunogenic coverage of ETEC strains. What C. jejuni capsule polysaccharide to conjugate is predicated primarily on epidemiological data suggesting pathogenicity of the strain providing the capsule polysaccharide. Although many *C. jejuni* strains exist, most are not pathogenic.

In the multipartite constructs listed in Table 4 and 5 the linker polypeptide, depending on the example construct, can comprise a four (4) amino acid sequence (tetrapeptide) or a tri-glycine. Also, as illustrated in FIG. 2, the subunits are interconnected and stabilized by donor strand complementation, which is denoted by "dsc". In this nomenclature, the fimbrial subunit derivation is also indicated. For example, in the construct "dsc_{16CstH}CstG-CstH-(G)-ntd₁₅dsc_{16CssA}CssA-CssB", the N-terminal CS3 subunit "CstG" is connected, via a linker, to the CS3 subunit "CstH", which is connected, via a linker, to a donor strand of 16 amino acids derived from "CstH." Similarly, the N-terminal CS6 subunit "CssB" is connected, via a linker, as illustrated in FIG. 2, to a 16 amino acid donor strand derived from "CssA." In this example, donor strand complementation of the "CssB" subunit is via a heterologous donor strand (i.e., derived from "CssA)."

In Table 4 and 5 the examples contain a "G" (i.e., glycine) to provide a "swivel." Also, in some examples, the N-terminal region of N-terminal CS6 subunit is deleted (delineated by "ntd") to avoid undesirable association with other CS6 subunits, as described above. It should be noted that, in addition to the examples illustrated in Table 4 or 5 (or Table 3), other combinations of major and minor subunits are contemplated utilizing the construct design illustrated in FIG. 2 and the fimbrial subunits of Table 2. In some sequences listed, a six (6) histidine (i.e., His₆) tag is inserted. The constructs can be designed to include the histidine (i.e., His₆) tag or designed without this tag region. Additionally, some sequences contain the signal peptide (designated "spd" in Table 2 and 3) region. Constructs can be constructed with or without this region, as well, which may be added to improve manufacturing efficiency of the multipartite fusion construct.

**Table 4**

| Fimbrial type (SEQ ID No. DNA/Protein) | Examples of CS3 containing constructs^{1, 2, 4, 5} |
|---|---|
| Class 5a/CS3 (6/7) | dsc_{14CfaB}-CfaE-CfaB-(G³)-ntd₁₈dsc_{16CstH}CstG-CstH |
| Class 5a/CS3 (8/9) | dsc_{14CsfA}CfaE-CfaB-CsuA2-CsfA-(G)-ntd₁₈dsc_{16CstH}CstG-CstH |
| Class 5b/CS3 (10/11) | dsc_{14csbA}CsbD-CsbA-ntd₁₅dsc_{14CooA}CooA-(G)-ntd₁₈dsc_{16CstH}CstG-CstH |
| Class 5c/CS3 (12/13) | dsc_{14CotA}CotD-CotA-(G)-ntd₁₈dsc_{16CstH}CstG-CstH |
| CS3/toxin fusion (36/37) | dsc_{16CstH}CstG-CstH-sCTA2 |
| LTB multimeric composition (38/39) | LTB₅ |
| CS3/CS6 (14/15) | dsc_{16CstH}CstG-CstH-(G)-ntd₁₅dsc_{16CstH}CssA-CssB |
| CS6/CS3 (34/35) | ntd₁₄dsc_{16cssB}CssB-CssA-(G)-ntd₁₈dsc_{16CstH}CstG-CstH |

| | |
|---|---|
| ¹ All combinations can include a histidine (i.e., His₆) at the C-terminal end. ² Subunits can be linked via either DNKQ or tri-glycine linker. ³ (G) refers to glycine residue introduced to provide a "swivel." ⁴ "ntd" refers to N-terminal deletion (excised from mature protein) with extent of deletion (i.e., amino acids) indicated. ⁵ "dsc" refers to span of N-terminal residues from donor β-strand, its amino acid length and its source. | |

**Table 5**

| Fimbrial type (SEQ ID No. DNA/Protein) | Examples of CS6 containing constructs |
|---|---|
| CS6/CS3 (34/35) | ntd₁₄dsc_{16CssB}CssB-CssA-(G)-ntd₁₈dsc_{16CstH}CstG-CstH |
| CS3/CS6 (32/33) | dsc₁₆CstG-CstH-(G)-ntd₁₄dsc_{16CssB}CssB-CssA |
| Class 5b/CS6 (28/29) | spd₁₉ dsc_{14CotA}CotD-CotA-(G)-ntd₁₄dsc_{16CssB}-CssB-CssA |
| Class 5b/CS6 (30/31) | dsc_{14CotA}CotD-CotA-(G)-ntd₁₄dsc_{16CssB}-CssB-CssA |
| Class5b/CS6 (24/25) | spd₁₉dsc_{15CsbA}CsbD-(GGG)-CsbA-(GGG)-ntd₁₄dsc_{14CooA}CooA-(G)-(GGG)-ntd₁₄dsc_{16CssB}CssB-CssA |
| Class 5b/CS6 (26/27) | dsc_{15CsbA}CsbD-(GGG)-CsbA-(GGG)-ntd₁₄dsc_{14CooA}CooA-(G)-(GGG)-ntd₁₄dsc_{16CssB}CssB-CssA |
| Class 5a/CS6 (16/17) | dsc_{14CfaB}CfaE-CfaB-(G)-ntd₁₆dsc_{16CssA}CssB-CssA |
| Class 5a/CS6 (113/114) | dsc_{14CfaB}CfaE-CfaB-(G)-ntd₁₆dsc_{16cssB}CssB-CssA |
| Class 5a/CS6 (18/19) | dsc_{14CfaB}CfaE-CfaB-(G)-ntd₁₆dsc_{16CssB}CssA-CssB |
| Class 5a/CS6 (111/112) | dsc_{14CfaB}CfaE-CfaB-(G)-ntd₁₆dsc_{16CssA}CssA-CssB |
| CS3/CS6 (101/102) | dsc_{16CssA}CssA-CssB-(G)-ntd₁₈dsc_{16CstH}CstG-CstH |
| Class 5a/CS6 (22/23) | dsc_{14CsfA}CfaE-CfaB-CsuA2-CsfA-(G)-ntd₁₄dscCssB-CssA |
| Class 5a/CS6 (20/21) | spd₂₂dsc_{14CsfA}CfaE-CfaB-CsuA2-CsfA-(G)-ntd₁₄dscCssB-CssA |
| CS6-chimera (40/41) | ntd₁₄dsc_{16CssB}CssB-CssA-sCTA2 |
| CS6-chimera (42/43) | ntd₁₅dsc_{16CssA}CssA-CssB-sCTA2 |

| | |
|---|---|
| All combinations can include a histidine (i.e., His₆) at the C-terminal end. ² Subunits can be linked via either DNKQ or tri-glycine (GGG) linker. In preferred embodiments, DNKQ is used, except where indicated with (GGG). ³ (G) refers to glycine residue introduced to provide a "swivel." ⁴ "spd" refers signal peptide. Number indicates number of amino acids. ⁵ "ntd" refers to N-terminal deletion (excised from mature protein) with extent of deletion (i.e., amino acids) indicated. ⁶ "dsc" refers to span of N-terminal residues from donor β-strand, its amino acid length and its source. | |

In another embodiment, recombinant polypeptide constructs can contain a C-terminal toxin A subunit, such as cholera toxin A2 (CTA) to form a chimeric molecule. In this embodiment, a full-length or truncated CTA2 is connected to CS6 or CS3 multimeric recombinant polypeptide construct, such as a CS6 or CS3 dimer.

Examples of these toxin constructs are illustrated in Table 4 and 5. In these constructs, the LTB gene and the CS3 or CS6 - toxin chimera are separately expressed. LTB, once expessed, would self assemble to form a pentameric structure. The ensuing LTB multimeric composition (i.e., LTB₅) and CS3 or CS6 -toxin chimera then non-covalently associate to form a holotoxin-like heterohexamer.

Although other examples are contemplated, the sequences of examples of illustrative chimeric constructs, containing a C-terminal toxin component, are illustrated in Table 4 (for CS3) and Table 5 (for CS6).

For CS3-chimeric molecules, one or more CS3 fimbrial subunits are connected, as in FIG. 1, via a polypeptide linker, preferably a tetrapeptide or triglycine. The C-terminal most CS3 fimbrial subunit is then connected to a donor β strand, via a polypeptide linker. The donor strand can be homologous or heterologous to the C-terminal fimbrial subunit. The donor strand is then connected to a toxin fragment, such as CTA2. The CS3-chimera example shown in Table 4, comprise the polypeptide sequence of SEQ ID No. 37, which is encoded by the DNA sequence of SEQ ID No. 36. In this example, the N-terminal fimbrial subunit is CstG with a pelB leader (22 amino acids) connected at its N-terminal end (see FIG. 13). However, different ordering of CS3 fimbrial subunit units is contemplated. Also, in this example, the CstH is connected, via a polypeptide linker, to a 16 amino acid donor strand derived from the N-terminal 16 amino acids of CstH, which is connected to an A2 toxin fragment (i.e., CTA2). In a preferred embodiment, LTB is also expressed. LTB comprises the amino acid sequence of SEQ ID No. 39 and is encoded by the nucleotide sequence of SEQ ID No. 38. Once expressed, the LTB sequence would self assemble into a pentamer and associate, non-covalently, with the CS3-chimera to form a hetero-hexameric holotoxin-like structure.

CS6 toxin chimera examples are also illustrated in Table 5. For CS6 chimeras, as in CS3, one or more CS6 fimbrial subunits are connected via a polypeptide linker, preferably a tetrapeptide or triglycine. The C-terminal most CS6 fimbrial subunit is then connected to a donor β strand, via a polypeptide linker. The donor strand can be homologous or heterologous to the C-terminal fimbrial subunit. The donor strand is then connected to a toxin component (e.g., CTA2). In a preferred embodiment, like for CS3, the chimera is co-expressed, with LTB, which self assembles into a pentamer to form a non-covalent association with the chimeric adhesion-toxoid fusion molecule.

Although many additional combinations are possible, in the examples shown in Table 5, the constructs are dimers of CS6 subunits, connected via a tetrapeptide linker, with the C-terminal fimbrial subunit connected, via a tetrapeptide linker to a donor β strand. The donor β strand can be homologous or heterologous to the C-terminal most fimbrial subunit. However, in the examples in Table 5 the donor strands are heterologous to the C-terminal fimbrial subunit. The donor strand is then connected to a cholera toxin A2 (CTA2) subunit. The polypeptide sequences of one of the examples is as in SEQ ID No. 43, which is encoded by the nucleotide sequence of SEQ ID Nos. 42. In this example, the N-terminal subunit is CssA, with the N-terminal 15 amino acids of the mature CssA sequence deleted. In this example, a pelB leader sequence (22 amino acids) was also added, which is illustrated in FIG. 14.

### Example 3: C. jejuni capsule polysaccharides

Recent development of a molecular CPS typing system re-enforced the strong correlation between CPS and Penner types (Poly, et al., J. Clin. Microbiol. 49: 1750 (2011)). Both Penner serotyping and molecular CPS typing have revealed the predominance of a handful of CPS types worldwide. Also, despite over 60 Penner serotypes having been identified, most Campylobacter diarrheal disease is caused by *C*. *jejuni* expressing only a limited number of serotypes. Therefore, only selected strains of C. jejuni, predicated on epidemiological studies, provides suitable candidate strains for development of vaccine compositions. However, despite the importance of this organism to human disease, there are no licensed vaccines against *C. jejuni.*

*C. jejuni* capsule polysaccharide (CPS) was extracted from C. jejuni strains selected based on their association with diarrheal disease. CPS from bacteria was extracted by hot water-phenol extraction for 2 h at 70 °C. The aqueous layer was dialyzed (1000 Da) against water followed by ultracentrifugation to separate the CPS from the LOS. The supernatant material containing the CPS was subjected to size-exclusion chromatography (Sephadex G50) for further purification to yield the intact CPSs. Monosaccharide composition was performed using a procedure amenable to the alditol acetate method (Chen, et al., Carbohydr. Res. 343: 1034 (2008)) with the alditol acetates being analyzed in a ThermoFinnigan POLARIS^{™}-Q (Thermo Fisher Scientific, Inc, Waltham, MA) gas chromatograph/mass spectrometer (GC/MS) using a DB-17 capillary column. The sugar linkage types were characterized by characterization of the permethylated alditol acetates by GC/MS as previously described (Chen, et al., Carbohydr. Res. 343: 1034 (2008)). The NMR experiments were performed on a Bruker 400 MHz spectrometer (Bruker Corporation, Billeria, MA) equipped with a Bruker cryo platform at 295 K with deuterated trimethylsilyl propanoic acid and orthophosphoric acid as external standards. The structures of important pathogenic *C. jejuni* capsule polysaccharides are shown in Table 6.

**Table 6**

| Capsule type | Polysaccharide structure |
|---|---|
| HS1 | 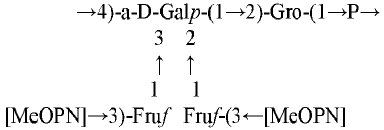 |
| HS44 | →4)-a-D-Gal*p*-(1→2)-Gro-(1→P→ |
| HS3 | →4)-[P→3]-alpha-D-Gal-(1→3)-[P→2/7]-6-d-alpha-D-ido-Hep-(1→; or |
| | →4)-[P→3]-alpha-D-Gal-(1→3)-[P→2]-L-glycero-alpha-D-ido-Hep-(1→ |
| | (where P represents O-methyl-phosphoramidate) |
| HS4/13/64 | →3)-6-deoxy-beta-D-ido-Heptose-(1→4)-beta-D-GlcNAc-(1→. |
| HS23/36 | [→3)-α-D-Gal-(1→2)-6d-α-D-*altro*-Me-Hep-(1→3)-β-D-GlcNAc-(1→]ₙ |
| HS15 | [→3)-α-Ara*f*-(1→3)-6-d-α-*gulo*-Hep*p*-(1→]ₙ |
| HS10 | 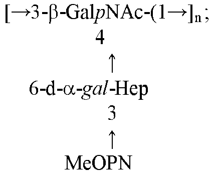 |
| HS13 | 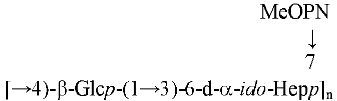 |
| HS13 | 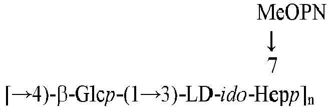 |
| HS2 | 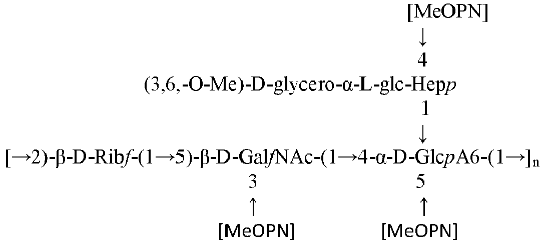 |

Additionally, the capsule polysaccharide from the HS5 strain of C. jejuni can be attached. HS 5 contains a complex of variations of polysaccharides. These include the following structures:

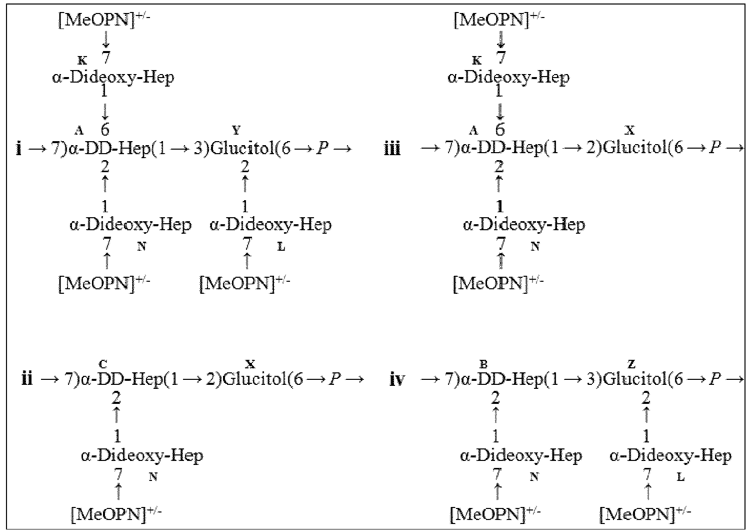

### Example 4: Induction of immune response by ETEC-Campylobacter capsule conjugates.

Induction of an immune response by the conjugates was evaluated. In these studies, BALB/c mice were immunized with escalating amounts of vaccines administered with alhydrogel^{®} (Sergent Adjuvants, Clifton, NJ). Mice received a total of two immunization at a 4-week interval.

The results of these studies is illustrated in FIG. 4. As shown in FIG. 4, two weeks following the first immunization, mice immunized with HS36-CfaEB (10 µg, 60 µg) and HS36-CfaE (60 µg) exhibited significant levels of serum IgG antibodies specific against HS36 CPS (p< 0.05) (see FIG. 4 (A)), comparared to pre-immune sera. Following two immunizations all groups of immunized animals had antibody levels that were significant increased (p<0.05) compared to levels observed after only one immunization. This effect was not dose dependent at the vaccine doses tested.

Furthermore, as illustrated in FIG. 4 (B), antibodies against CfaE was also determined by ELISA. As shown in FIG. 4(B), all mice immunized with the conjugate vaccine possessed significant levels of anti-CfaE IgG (p<0.05) for CPS - CfaE or CPS CfaEB. No dose dependent effects were observed and all groups displayed similar levels of CfaE-specific IgG.

The data shown in FIG. 4 illustrates that the conjugate vaccine comprising an ETEC adhesin-based carrier protein conjugated to a *C. jejuni* CPS is capable of inducing an immune response against both bacterial components, i.e., *C. jejuni* CPS and ETEC CfaE.

To determine the levels of functional anti-adhesive antibody generated by HS36 conjugate vaccines, serum samples were tested by hemagglutination inhibition assay (HAI) assay in order to measure functional anti-adhesive antibodies present in the immune mouse serum.

The HAI assays were conducted by evaluating samples from each animal. The samples were initially diluted 1:8, then diluted two-fold over a wide range of dilutions. Each serum dilution was incubated with an equal volume of CFA/I⁺ ETEC bacteria (strain H10407), which further diluted the serum 1:2. The final lowest dilution tested dwas 1:16, which was the limit of detection (LOD). The pre-incubated mixture was subsequently mixed with bovine erythrocytes in the presence of 0.5% mannose in U-bottom 96-well plates. In the absence of anti-adhesive antibodies, the erythrocytes formed visible agglutinated "buttons" of cells. In the presence of anti-adheisve antibodies, agglutination was inhibited. The HAI titer was the highest serial dilution that completely inhibited agglutination. If there was not detectable inhibition at the lowest serum dilution of 1:16, the samples were assigned a value of one-half of the detection limit (i.e., 8) for computational purposes.

The results of the HAI analysis are illustrated in FIG. 5. Prior to immunization, pooled serum contained HAI titers below the the assay's level of detection. Following immunization, all groups of mice displayed significantly (p<0.05) higher levels of anti-adhesive antibodies in their sera compared to pre-immune titers. Genrally, mice immunized with HS36-CfaEB conjugate vaccine exhibited higher HAI titers. However, the only significance difference (P<0.05) observed was between mice immunized with an HS36-CfaEB (60 µg) and HS36-CfaE (10 µg).

### Example 5: Immune response against multiple MeOPN-6-Gal

### Synthesis of polysaccharide construct

A polysaccharide constructed was synthesized as shown in FIG. 6. Starting from a previously reported compound 4-methoxyphenyl-α-D-galactopyranoside (see also FIG. 7, structure 1) (Comfort, et al., Biochem. 46: 3319-3330 (2007)), trityl group was selectively introduced to C-6. Originally, benzoylation was performed on compound (FIG. 7, structure 2), however extensive migration observed during the introduction of MeOPN lead us to look for a more suitable protecting group. Therefore, allyl groups were selected to protect the C-2, C-3 and C-4 positions which were resistant to migration. Allyl groups were later deprotected with catalytic hydrogenolysis which proved to be compatible with the MeOPN modification.

As shown in FIG. 6 and FIG. 7, after allyl groups were installed, an aminopentanyl linker was introduced to the anomeric position as a site for conjugation. Starting from galactoside (FIG. 7, structure 3), 4-methoxyphenyl group (OMP) was first removed with cerium ammonium nitrate (CAN). The corresponding hemiacetal was then converted into trichloroacetimidate donor . 5-Amino-*N*-phthalimido-pentanyl linker was then introduced with TMSOTf as activator at 0 °C. Compound 5 (FIG. 7) was collected with 65% as the β anomer and 29% as the α anomer. The removal of trityl group gave a free 6-hydroxyl group for modification.

The strategy for the introduction of MeOPN group was inspired by a similar reaction initially proposed by C. Mara et al, Bioorg. Med. Chem. Lett. 6180-6183 (2011). Compound 6 (FIG. 6 and FIG. 7) was treated with commercially available methyl dichlorophosphate in the presence of triethyl amine, followed by ammonolysis. Due to the chirality nature of the newly introduced MeOPN (R and S), product 7 (FIG. 7, structure 7) was collected as a mixture of two diastereoisomers. ¹H NMR was able to confirm that product 7 (FIG. 7) was indeed a 1:1 mixture of two diastereoisomers, revealing two sets of signals throughout the spectrum, such can be seen for anomeric and O-Me signals. The reaction yielded a mixture of side products, the most abundant being the O-Me group being replaced by a second NH₂, accounting for the poor yield of this reaction.

Allyl and phthlimido protecting groups were removed with palladium (II) chloride and hydrazine respectively, generating product 9 (FIG. 7, structure 9). Similar to compound 7 (FIG. 7), a mixture of diastereoisomers is apparent in NMR. Although not optically pure, the ³¹P NMR result agrees with native MeOPN-containing polysaccharides, having a phosphorous signals around 14 ppm.^{3 1}H-³¹P HMBC NMR experiment was able to confirm that the MeOPN was introduced to the O-6 position, showing correlation signal with O-Me as well as the H-6 signals.

### Induction of immunity against MeOPN-6-Gal

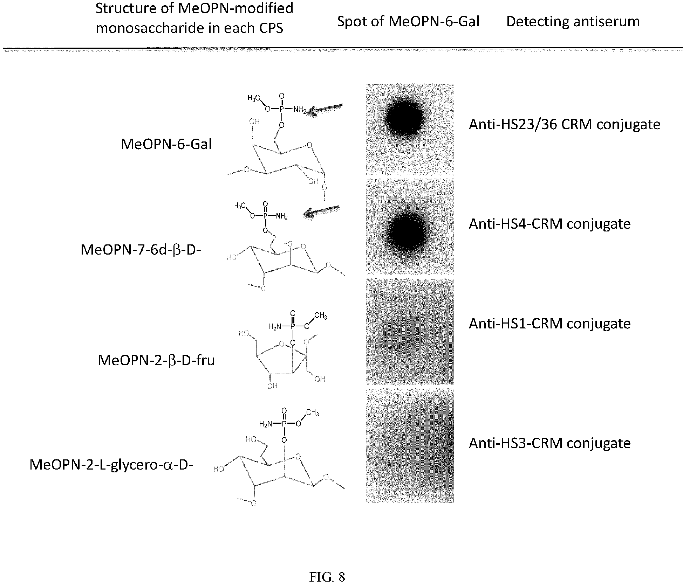

In one embodiment, galactose modified at the 6 carbon with O-methyl phosphoramidate (MeOPN-6-Gal) is used to induce immunity against multiple C. jejuni strains, even those strains not expressing MeOPN-6-Gal. As illustrated in FIG. 8, the monosaccharide construct MeOPN-6-Gal was recognized by antibody against capsule polysaccharide isolated from HS23/36, conjugated to CRM₁₉₇. Unexpectedly, antibody against polysaccharide from HS4, conjugated to CRM₁₉₇, also elicited an equivalent response, as anti-HS23/36 CRM₁₉₇ conjugate, against MeOPN-6-Gal. Also, anti-HSl-CRM₁₉₇, also reacted to MeOPN-6-Gal, although to a somewhat less extent.

The strong cross-reactivity with MeOPON-6-Gal exhibited against HS23/36 and HS4 antibody may be explained by the the fact that MeOPN-6-Gal share epitopic structures with HS23/36 and HS4 capsule polysaccharides. One explanation may be that the MeOPN group in both HS23/36 and HS4 is to a primary hydroxyl. The cross reaction of MeOPN-6-Gal (HS23/36) with HS4, which contains MeOPN-7-6d-β-D-*ido*-Heptose, was unexpected, but may be due to the linkage of MeOPN to primary hydroxyl positions on both sugars. This feature is illustrated in FIG 8 by the arrow.

### Example 6: Immunogenic composition against C. jejuni and enterotoxigenic Escherichia coli (ETEC) using a combined C. jejuni capsule/ETEC construct

A synthetic conjugate vaccine strategy can be developed to protect against multiple enteric pathogens. Most efforts at development of vaccines against bacterial enteric pathogens are limited to a specific pathogen. The ability to combine vaccines against multiple, antigenically variable pathogens in a single, multi-valent, injectable vaccine would greatly simplify approaches to prevent acquisition and transmission of these pathogens worldwide. Globally, ETEC and *C. jejuni* are among the leading causes of bacterial diarrheal disease. In addition CJ has been causally linked to several serious sequelae including Guillain Barre Syndrome, irritable bowel syndrome, and reactive arthritis. Moreover, recent studies have indicated an association between CJ infections and malnutrition and growth stunting in young children in resource-limited settings.

Using conventional methods, we have developed conjugate vaccines containing CJ polysaccharide capsules that have proven to be immunogenic in multiple animal species and to confer protection against *C. jejuni* diarrhea in NHP. The newer synthetic approach is based on recent data that the immunodominant epitope on CJ polysaccharide capsule conjugate vaccines is the MeOPN modification found on different sugars in different capsule types.

Therefore, an immunogenic platform against both *C. jejuni* and ETEC can be created by linking synthetic MeOPN-sugars to different ETEC protein antigens. The approach could also be extended to include *Shigella* lipopolysaccharides (synthetic or detoxified) conjugated to ETEC proteins. Thus, this platform could form the basis of a multivalent vaccine against three major bacterial diarrheal pathogens. Conjugation can also serve as a protein carrier to enhance immunogenicity of the Campylobacter construct.

It is envisioned to conjugate the construct of Examples 3 - 5 to an ETEC construct. The overall method of conjugating includes oxidizing *C. jejuni* CPS, for example, with NaIO₄ in sodium acetate (pH 4.0). Oxidized CPSs were desalted with a 5 kDa cutoff membrane by stirred ultrafiltration, which is subsequently lypholized. ETEC proteins are then added. The stoichiometery protein to CPS can vary, however, a typical ratio is 1:2 protein to CPS by mass. The concentration of components can be by any method. However, for example, polysaccharide concentration was determined by antrhone assay and protein concentration was determined by Pierce 660 protein assay or the BCA assay. NaCNBH₃ is then added. The conjugates can then be subsequently desalted by ultrafiltration and lyophilized. CPS, ETEC proteins and conjugates were analyzed, for example by SEC-HPLC or by SDS polyacrylaminde gel electrophoresis (PAGE), or other methods.

### Example 7: Non-human primate response

The immunogenicity of CfaE-HS23/36 and CfaEB-HS23/36 conjugates was observed in mice, as well as induction of hemagglutination inhibition (HAI) titers against Cfal in mice. The amino acid sequence of the dscCfaE construct used is SEQ ID No. 138 (nucleotide sequence is SEQ ID No. 139). The dsc₁₉CfaE amino acid sequence is SEQ ID No. 143 (nucleotide sequence is SEQ ID No. 142). The amino acid sequence for dsc₁₉CfaEB is SEQ ID No. 141 (nucleotide sequence is SEQ ID No. 140).

The CfaEB-HS23/36 conjugate was down-selected in order to proceed to studies in *Aotus nancymaae.* This non-human primate (NHP) model was selected because it has been used as a diarrheal disease model for both ETEC and *C. jejuni.* We synthesized a lot of the CfaEB-HS23/36 vaccine that was sufficient in size for three NHP studies by reductive amination. The first such study, which is the only one that has been completed, was a dose finding study followed by a C. jejuni challenge.

The design of this NHP study is shown in Table 7. Animals (6 per group) were immunized three times at days 0, 42, and 84. The CfaEB-HS23/36 vaccine was given subcutaneously at either 0.5 ug or 3.5 ug polysaccharide (PS) adjuvanted with aluminum hydroxide. The ratio of PS to protein in the vaccine was roughly 1:1 so this was equivalent to 0.5 or 3.5 ug of CfaEB per dose. The 3.5 ug dose was also given intradermally (ID) with poly-IC as adjuvant. This was done to bridge to previous work done using ID immunizations with CfaEB alone. Similarly, another group was given HS23/36-CRM197 subcutaneously to bridge to previous work with the same capsule conjugated to another protein. Finally, the control group was immunized with PBS. On day 148 the animals were all challenged with 4 x 10¹¹ CFU of CG8421, an HS23/36 strain.

**Table 7: Design of NHP study**

| **Group** | **Route** | **CfaEB-CPS** | **CPS-CRM197** | **Alum (ug)** | **Poly IC (ug)** | **PBS** |
|---|---|---|---|---|---|---|
| 1 | SC | 0.5 | - | 300 | - | - |
| 2 | SC | 3.5 | - | 300 | - | - |
| 3 | ID | 3.5 | - | - | 100 | - |
| 4 | SC | - | 3.5 | 300 | - | - |
| 5 | SC | - | - | - | - | + |

Animals were observed for diarrheal disease daily for 10 days following challenge. Diarrhea was defined as two or more days of consecutive of stools that were ≥ grade 3. The results are summarized in Table 8. Only 3/5 animals in the PBS group developed diarrhea for an attack rate of 60%. Note that one animal was eliminated because it developed diarrhea prior to challenge. The mean time to onset of disease in this negative control group was 2.3 days and the mean duration of illness was 5.3 days. The attack rate in the animals immunized with the HS23/36-CRM197 vaccine was 33% (2/6), with a mean onset of disease of 2 days and a mean duration of illness of 4 days (45% efficacy). Animals that were immunized with CfaEB-HS23/36 intradermally with poly IC also showed an attack rate of 33% with a mean onset of 1.5 days and a duration of two days (45% efficacy). The animals immunized subcutaneously with CfaEB-CPS showed between 67-100% efficacy against diarrheal disease. The attack rate in the group immunized with 0.5 ug of the vaccine was 0 (0/5, with one animal that vomited after challenge being eliminated) and the attack rate in the group immunized with 3.5 ug of the vaccine was 20% (1/5, with one animal being eliminated due to diarrhea prior to challenge). The single animal in this group that did develop diarrhea had a later onset of disease (day 9). There were no significant differences among the control group and any of the immunized animals due to the small numbers of animals per group.

**Table 8. Results of challenge with C. jejuni CG8421.**

| **Group** | **Vaccine** | **#ill/total** | **Attack rate (%)** | **Mean days to onset of diarrhea (range)** | **Mean days of illness (range)** | **Protective efficacy** |
|---|---|---|---|---|---|---|
| 1 | CfaEB-CPS (0.5 ug) + alum | 0/5^{∗} | 0 | 0 | 0 | 100 |
| 2 | CfaEB-CPS (3.5 ug) + alum | 1/5^{∗∗} | 20 | 9 | 2 | 67 |
| 3 | CfaEB-CPS (3.5 ug) + poly IC | 2/6 | 33 | 1.5 (1-4) | 2 (2-4) | 45 |
| 4 | CRM-CPS + alum | 2/6 | 33 | 2 (1-3) | 4 (2-6) | 45 |
| 5 | PBS | 3/5^{∗∗} | 60 | 2.3 (1-6) | 5.3 (2-10) | - |

| | | | | | | |
|---|---|---|---|---|---|---|
| ^{∗} One animal vomited after challenge and was excluded ^{∗∗} Animals were excluded from analyses due to diarrheal onset prior to challenge | | | | | | |

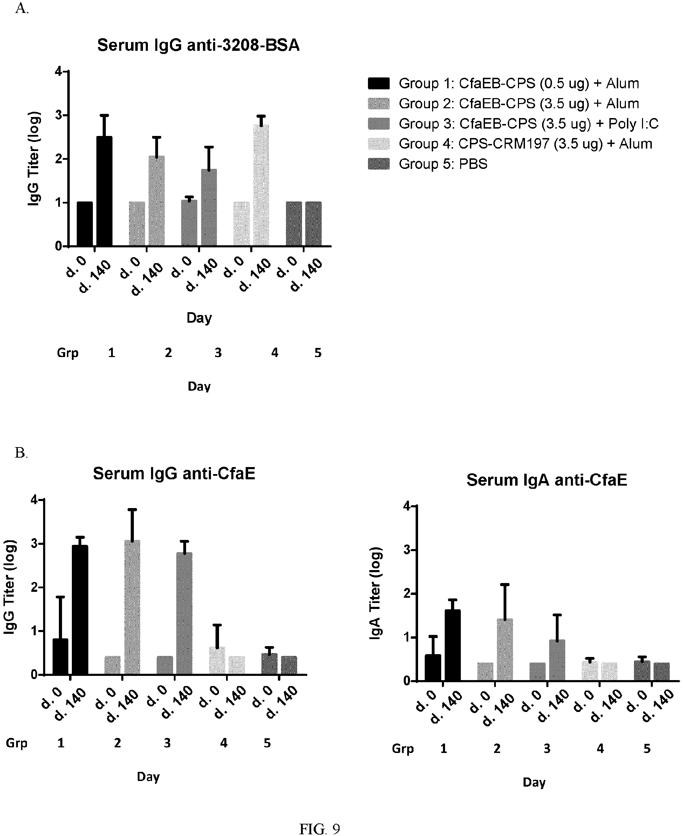

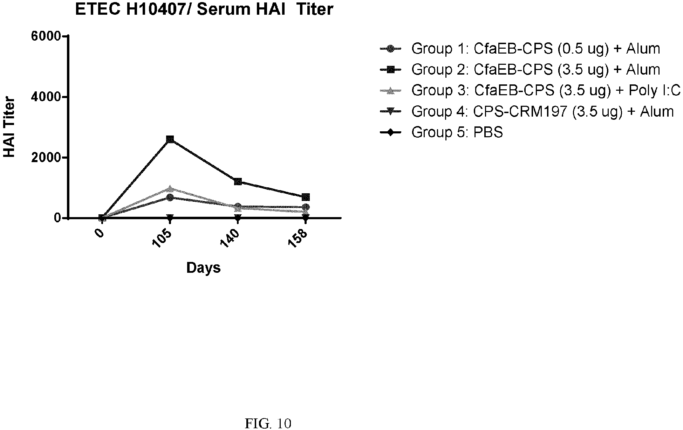

Serology results are shown in Fig. 9. Immune responses to CPS and to CfaE were measured by ELISA. Animals in groups 1, 2 and 3 displayed IgG responses to both antigens and and IgA response to CfaE. Hemagglutination inhibition (HAI) titers against ETEC strain H10407 expressing CfaI fimbriae were determined and are shown in Fig. 10. The results indicate that HAI titers were detected in animals in groups 1, 2 and 3, with group 2 showing the highest titers.

### Example 8: Synthesis and immunogenicity of additional combinations ofETEC-Campylobacter capsule conjugates

CssBA-HS3 vaccine. CssBA is a recombinant form of the two subunits of CS6 that are fused together. This protein was conjugated to capsule from an HS3 strain by TEMPO oxidation. The conjugates were analyzed by SDS-PAGE and immunoblotting. Purified CssBA has a predicted Mr of 31.8 kDa. The conjugate of CssBA-HS3 CPS runs as two bands, one slightly smaller than CssBA and one that runs at approximately 60 kDa. The bands in the conjugate react with both anti-CssBA antiserum and antibodies to whole cells of HS3, indicating that polysaccharide has been conjugated to the protein.

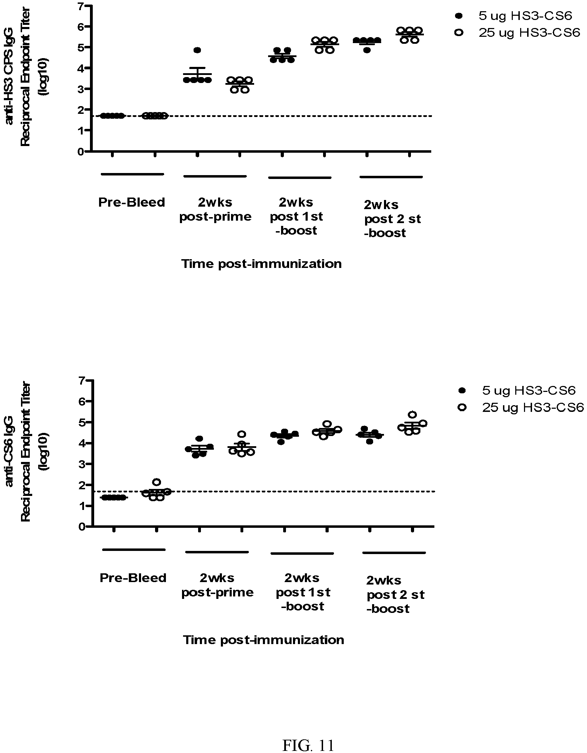

Mice were immunized subcutaneously with three doses of the vaccine given at 4 week intervals. Doses were 5 ug by weight or 25 ug by weight. Animals were bled at day 0 and two weeks after each immunization and the response to CssBA and to CPS were determined by ELISA. The results, shown in Fig. 11, indicate that there was a robust response to both the protein and the polysaccharide at both doses.

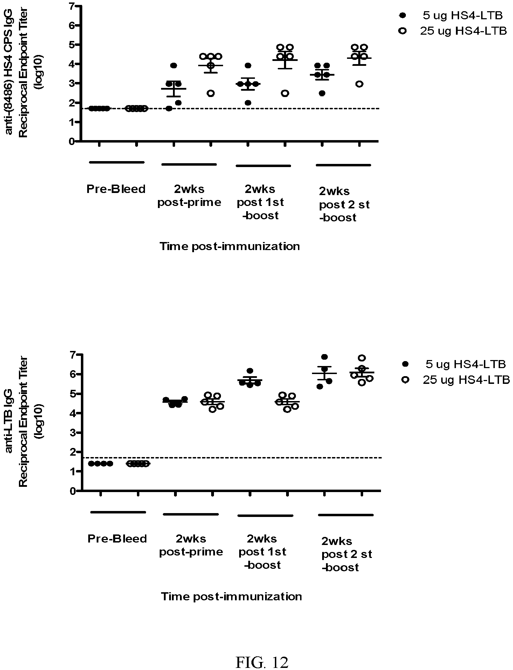

LTB-HS4 vaccine. LTB is the binding component of the heat labile enterotoxin of ETEC. Recombinant LTB, which is not toxic, was conjugated to the polysaccharide capsule of an HS4 strain by reductive amination. The conjugate was analyzed by immunoblotting as shown in Fig. 12. Immunodetection with anti-LTB antiserum revealed a single band for LTB at approximately 10 kDa. The conjugate contained 4 major bands ranging from ~20kDa->75kDa that were reactive with both anti-LTB and anti-HS4 antiserum, indicating successful conjugation.

Mice were immunized with three doses of either 5 or 25 ug (by weight) of the LTB-HS4 conjugate subcutaneously at 4 week intervals and the serum immune response was determined. The results, shown in Fig. 12, indicate that there was a robust immune response to both the HS4 capsule and to LTB at both doses.

### Example 9: Conjugation to Shigella lipopolysaccharide (LPS)

There are four species of Shigella, a human pathogen cause diseases such as diarrhea and bacilliary dysentaery: *Shigella dysenteriae, Shigella flexneri*, *Shigella boydii* and *Shigella son*nei are important enteropathogens Strains of *Shigella* spp. Express long-chain lipopolysaccharides. The chemical structures for many strains has been determined (see Liu, et al., FEMS Microbiol. Rev. 32: 627-653 (2008)).

An object of this invention, is a *Shigella* LPS-ETEC construct. The construct comprises an ETEC construct, as the above examples, conjugated, to a *Shigella* LPS, as an alternative or in addition to *C. jejuni* capsule polysaccharide. It is envisioned that any of the *Shigella* spp. can be conjugated to the ETEC construct. As an example, the *Shigella flexneri* 2a LPS is illustrated, as a potential LPS structure that can be conjugated to an ETEC construct, as follows:

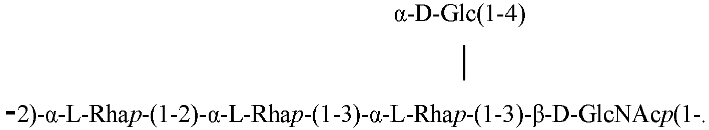

## Claims

1. A multi-agent immunogenic construct, comprising a *Shigella* lipopolysaccharide conjugated to a protein carrier, wherein said protein carrier comprises an enterotoxigenic *Escherichia coli* (ETEC) recombinant polypeptide construct.

2. The multi-agent immunogenic construct of claim 1, wherein said ETEC recombinant polypeptide construct comprises an ETEC minor or major subunit connected at the C-terminal to the N-terminal of one or more ETEC major fimbrial subunits, thereof, of the same ETEC fimbrial type, via a polypeptide linker, and wherein each of the one or more ETEC major fimbrial subunits contain a donor β strand at their N-terminus and each of the major ETEC fimbrial subunits are connected to each other via a polypeptide linker wherein the C-terminal of the one or more ETEC fimbrial subunit is connected, via a linker, to a donor β strand derived from a major ETEC fimbrial subunit of the same ETEC fimbrial type as the one or more major ETEC fimbrial subunits, and_wherein the ETEC recombinant polypeptide construct can contain a C-terminal histidine tag at the C-terminus.

3. The multi-agent immunogenic construct of claim 1, wherein the multi-agent immunogenic construct comprises *Campylobacter jejuni* capsule polysaccharide in addition to *Shigella* lipopolysaccharide.

4. The multi-agent immunogenic construct of claim 1, wherein said molar ratio of *Shigella* spp. lipopolysaccharide to ETEC recombinant protein carrier is 1:1 to 5:1.

5. The multi-agent immunogenic construct of claim 2, wherein said ETEC minor or major fimbrial subunits are derived from ETEC strains selected from the group consisting of Class 5, CS3 and CS6, preferably wherein said ETEC minor fimbrial subunit is selected from the group consisting of CfaE, CsfD, CsuD, CooD, CsbD, CosD, CsdD, CotD and wherein said major subunit is selected from the group consisting of CfaB, CsfA, CsuA2, CooA, CsdA, CosA, CsbA, CotA, CstG, CstH, CssA, and CssB, or derivatives, thereof.

6. The multi-agent immunogenic construct of claim 2, wherein the *Escherichia coli* recombinant polypeptide construct comprises an amino acid sequence selected from the group consisting of SEQ ID Nos. 7, 9, 11, 13, 15, 17, 19, 21, 23, 25, 27, 29, 31, 33, 35, 37, 41, 43, 102, 103, 105, 107, 109, 112, 114, 138, 141 and 143.

7. The multi-agent immunogenic construct of claim 2, wherein the *Escherichia coli* recombinant polypeptide construct is encoded by the nucleotide sequence of SEQ ID Nos. 6, 8, 10, 12, 14, 16, 18, 20, 22, 24, 26, 28, 30, 32, 34, 36, 40, 42, 101, 104, 106, 108, 110, 111, 113, 139, 140, and 142.

8. The multi-agent immunogenic construct of claim 3, wherein said *Campylobacter jejuni* polysaccharide is a repeating trisaccharide structure having the formula selected from the group consist of:
[→3)-a-D-Gal-(1→2)-6d-a-D-*altro*-Me-Hep-(1→3)-β-D-GlcNAc-(1→]ₙ ;
[→3)-β-6-deoxy-D-ido-Heptose (1→4)-β-D-GlcNAc-(1→]ₙ ;
[→3)-α-Ara*f*-(→3)-6-d-α-*gulo*-Hep*p*-(1→]ₙ ; 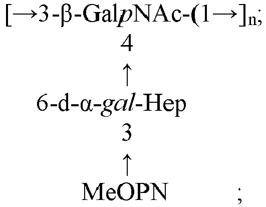 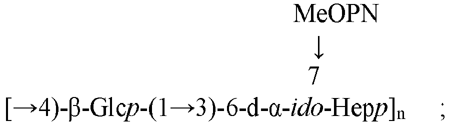 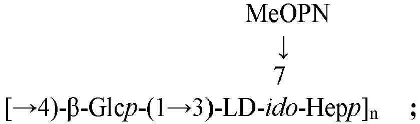 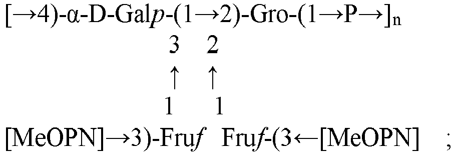
[→2)-D-*glycero-*α-D-manno-Hep*p*-(1-4)-α-D-Glc*p*-(1→]ₙ; 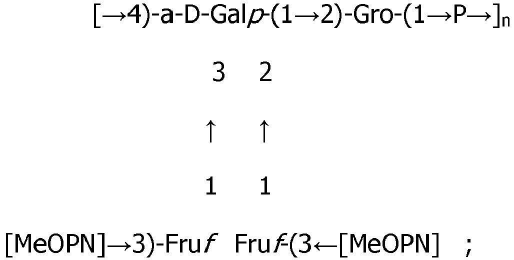
[→3)-L-beta-D-ido-Hep-(1->4)-beta-D-Glc-(1→]ₙ, with non-stoichiometric substitution of O-methyl-phosphoramidate at position 2 of L-glycero-beta-D-ido-heptose;
[→3)-6d-beta-D-ido-Hep-(1->4)-beta-D-Glc-(1→]ₙ, derived from HS13, with non-stoichiometric substitution of O-methyl-phosphoramidate at position 2 or/and 7 of 6-deoxy-beta -D-ido-heptose;
[→3)-L-beta-D-ido-Hep-(1->4)-beta-D-Glc-(1→]ₙ ;
[→3)-L-alpha-D-ido-Hep-(1->4)-alpha-Gal-(1→]ₙ, with non-stoichiomoetric substitution O-methyl-phosphoramidate at position 2 of 6-deoxy-alpha-D-ido-heptose; 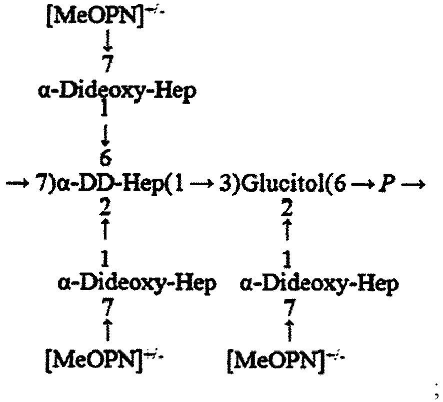 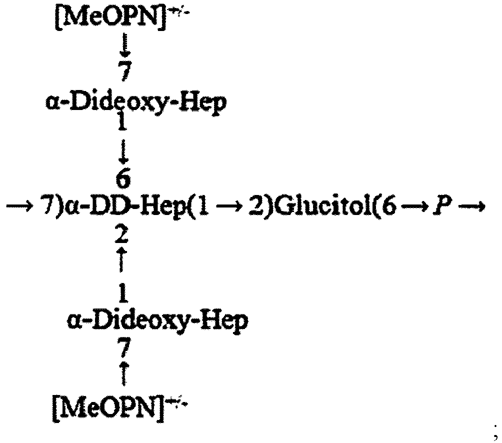 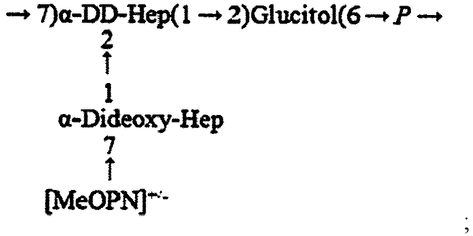 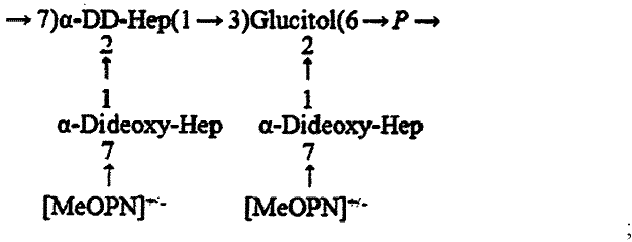 and
[→3)-6d-alpha-D-ido-Hep-(1->4)-alpha-Gal-(1→]ₙ, derived from HS3, HS13 and HS50 with non-stoichiometric substititution of O-methyl-phosphoramidate at position 2 of L-glycero-alpha-D-ido-heptose, wherein "n" is 1 to 100.

9. The multi-agent immunogenic construct of claim 1, wherein said Shigella lipopolysaccharide has the structure:

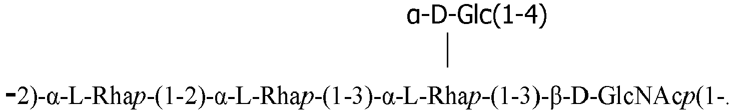

10. The multi-agent immunogenic construct of claim 2, wherein (i) said donor β strand contains 12 to 16 amino acids, (ii) said N-terminus of said minor or major subunit contains an 18-22 amino acid signal peptide, (iii) the amino acid sequence of said polypeptide linker is the amino acid sequence of SEQ ID No. 5 or a tri-glyicine, or (iv) one or more major subunits contain a deletion of the 14 to 18 N-terminal amino acids.

11. The multi-agent immunogenic construct of claim 5, wherein said *Escherichia coli* fimbrial minor subunit is selected from the group consisting of CfaE, CsfD, CsuD, CooD, CsbD, CosD, CsdD, CotD and wherein said major subunit is selected from the group consisting of CfaB, CsfA, CsuA2, CooA, CsdA, CosA, CsbA, CotA, CstG, CstH, CssA, and CssB, or immunogenic fragements or derivatives, thereof, and wherein said amino acid sequence of said *Escherichia coli* fimbrial minor subunit is selected from the group consisting of SEQ ID Nos. 45, 46, 51, 52, 57, 58, 65, 71, 75, 79, 83, 88, 90, 93, 95, and 97, or derivatives thereof, and wherein said amino acid sequence of said *Escherichia coli* fimbrial major subunit is selected from the group consisting of SEQ ID Nos. 2, 4, 48, 49, 54, 55, 60, 61, 63, 67, 69, 73, 77, 81, 85, 87, 89, 91, 92, 94, 96, 98, 99, 101, 135, and 137, or derivatives thereof.

12. A multi-agent immunogenic construct of claim 2 for use in a method of inducing an immune response against one or more enterobacteria selected from the group consisting of *C. jejuni* strains, *Escherichia coli,* and *Shigella,* comprising the steps:
a. administering the multi-agent immunogenic composition of claim 2 at a dose range of 0.1 µg to 10 mg per dose;
b. administering a boosting dose of said capsule polysaccharide composition at a dose range of 0.1 µg to 10 mg per dose.

13. The method of claim 12, wherein the multi-agent immunogenic construct comprises *Campylobacter jejuni* capsule polysaccharide in addition to *Shigella* lipopolysaccharide, and wherein said *Campylobacter jejuni* capsule polysaccharide comprises the polysaccharide structures of claim 8.

14. The method of claim 12, wherein said multi-agent immunogenic composition comprises the construct of claim 3.

15. The method of claim 12, wherein :
(i) said *Escherichia coli* enterotoxigenic recombinant polypeptide construct wherein said *Escherichia coli* fimbrial minor subunit is selected from the group consisting of SEQ ID Nos. 45, 46, 51, 52, 57, 58, 65, 71, 75, 79, 83, 88, 90, 93, 95, and 97, or derivatives thereof, and wherein said amino acid sequence of said *Escherichia coli* fimbrial major subunit is selected from the group consisting of SEQ ID Nos. 2, 4, 48, 49, 54, 55, 60, 61, 63, 67, 69, 73, 77, 81, 85, 87, 89, 91, 92, 94, 96, 98, 99, 101, 135, and 137, or derivatives thereof,
(ii) the *Escherichia coli* recombinant polypeptide construct comprises an amino acid sequence selected from the group consisting of SEQ ID Nos. 7, 9, 11, 13, 15, 17, 19, 21, 23, 25, 27, 29, 31, 33, 35, 37, 41, 43, 102, 103, 105, 107, 109, 112, 114, 138, 141, and 143,
(iii) the *Escherichia coli* recombinant polypeptide construct is encoded by the nucleotide sequence of SEQ ID Nos. 6, 8, 10, 12, 14, 16, 18, 20, 22, 24, 26, 28, 30, 32, 34, 36, 40, 42, 101, 104, 106, 108, 110, 111, 113, 139, 140, and 142,
(iv) said donor β strand contains 12 to 16 amino acids,
(v) said N-terminus of said minor or major subunit contains an 18-22 amino acid signal peptide,
(vi) the amino acid sequence of said polypeptide linker is the amino acid sequence of SEQ ID No. 5 or a tri-glyicine, or
(vii) one or more major subunits contain a deletion of the 14 to 18 N-terminal amino acids.
